# EUROPEAN PATENT APPLICATION

(11) **EP 4 578 454 A1**
(43) Date of publication of application: **02.07.2025**
(21) Application number: 23220484.2
(22) Date of filing: 28.12.2023
(51) Int. Cl.: A61K 35/19, A61K 9/00, A61K 45/06, A61P 29/00, A61P 37/02

(54) **COMPOSITIONS COMPRISING PLATELET DERIVED EXTRACELLULAR VESICLES**

(71) Applicant: Lysatpharma GmbH, 07607 Eisenberg (DE)
(72) Inventor: de Miroschedji, Kyra, 07743 Jena (DE)
(74) Representative: DTS Patent- und Rechtsanwälte PartmbB

(57) **Abstract**

The invention relates to a composition comprising platelet derived extracellular vesicles, e.g., *human* platelet lysate derived extracellular vesicles, for use in the prevention and/or treatment of an immune disorder and/or of an inflammatory disorder in a subject, wherein the composition is administered to the subject.

## Description

The present invention relates to compositions for use in the prevention and/or treatment of an immune disorder and/or of an inflammatory disorder in a subject.

US 2020/0054683 A1 describes the use of exosomes in a method of promoting, restoring or enhancing homeostasis in an individual suffering from graft versus host disease (GVHD) or epidermolysis bullosa (EB). This document is strictly focused on the use of mesenchymal stem cell exosomes.

US 2014/0031256 A1 describes a method of detecting a therapeutic exosome, the method comprising detecting an activity of an exosome. The activity may be selected from a group of activities. The group of activities comprises *inter alia* immunodulatory activity. This document further describes a method comprising a step of isolating an exosome from a mesenchymal stem cell conditioned medium.

WO 2021/226108 A1 describes pharmaceutical or cosmetic compositions comprising secretomes, for example secreted proteins from stem cells, and uses thereof.

EP 3 468 568 B1 describes a pharmaceutical preparation comprising a fraction that is enriched for *human* platelet lysate derived extracellular vesicles for use in medicine, e.g., for use in the prevention and/or treatment of inflammatory driven diseases, neurodegenerative diseases, immune/autoimmune diseases, cardiovascular diseases, dermatologic diseases, infectious diseases, transplant rejections, stroke, ischemia or graft-versus-host disease.

Although much progress has been made in the prevention and/or treatment of immune disorders and/or of inflammatory disorders, there is an urgent need for efficient therapies. This applies in particular for patients who do not respond well to established medication regimes or who experience disproportionately severe side-effects. In this regard, "efficient therapies" may refer in particular at least one of the following:
- an overall amount of therapeutic and/or pharmaceutically active composition to be administered to a patient in order to reach a desired immunosuppressive and/or anti-inflammatory effect,
- an overall efficacy and/or selectivity in terms of desired preventive or therapeutic effects per undesired side-effect(s), and/or
- resources required for the preparation of a dose of a therapeutic and/or pharmaceutically active composition.

A problem underlying the invention can be seen in the provision of a composition for use in the prevention and/or treatment of an immune disorder and/or of an inflammatory disorder in a subject, which composition is efficient. It may, for example, be efficient a) with regard to the amount of drug required in order to reach a desired immunosuppressive and/or anti-inflammatory pharmaceutical effect, and/or b) with regard to suppressing or avoiding undesired side-effects.

This problem is solved by a composition comprising platelet derived extracellular vesicles for use in the prevention and/or treatment of an immune disorder and/or of an inflammatory disorder in a subject, wherein the composition is administered to the subject, wherein
- the composition is administered to the subject in combination with at least one drug, wherein the at least one drug is selected from the group of immunosuppressive and/or anti-inflammatory drugs
   and/or
- the immune disorder and/or inflammatory disorder is selected from the group of
   - a graft-versus-host disease (GvHD),
   - a transplant rejection, e.g., a rejection of a transplanted organ or tissue, in particular a host-versus-graft disease (HvGD),
   - autoimmune disorders, e.g.,
      - autoimmune intestinal or bowel disorders, e.g., Crohn's disease or ulcerative colitis,
      - autoimmune skin and/or ocular disorders, e.g., psoriasis, vitiligo, atopic dermatitis, uveitis, in particular autoimmune uveitis, for example refractory autoimmune uveitis,
      - autoimmune musculoskeletal disorders, e.g., arthritis, in particular rheumatoid arthritis (RA) or juvenile idiopathic arthritis (JIA), ankylosing spondylitis (Bekhterev's disease), autoimmune myositis, autoimmune polychondritis,
      - autoimmune connective tissue diseases (autoimmune collagenoses), e.g., Sjögren's syndrome (SjS, SS), lupus erythematosus, in particular systemic lupus erythematosus (SLE), systemic sclerosis, in particular systemic sclerosis-associated interstitial lung disease (SSc-ILD), autoimmune vasculitis,
      - neuroinflammation-associated neurodegenerative disorders, e.g., Alzheimer's disease (AD), Parkinson's disease (PD), amyotrophic lateral sclerosis (ALS), neurodegenerative disorders, multiple sclerosis (MS),
      - other autoimmune disorders, e.g., autoimmune hepatitis, type 1 diabetes mellitus, lupus nephritis, myasthenia gravis, Grave's disease, and
   - low-grade inflammation and chronic low-grade inflammation, disorders associated with low-grade inflammation and chronic low-grade inflammation, e.g., myalgic encephalomyelitis (ME), chronic fatigue syndrome (CFS), myalgic encephalomyelitis/chronic fatigue syndrome (ME/CFS), post-viral fatigue syndrome (PVFS), post-COVID syndrome (PCS, long COVID), adverse reactions after COVID-19 vaccination (Post Vac).

A first aspect of the invention relates to a composition comprising platelet derived extracellular vesicles for use in the prevention and/or treatment of an immune disorder and/or of an inflammatory disorder in a subject, wherein the composition is administered to the subject,
wherein
- the composition is administered to the subject in combination with at least one drug, wherein the at least one drug is selected from the group of immunosuppressive and/or anti-inflammatory drugs
   and
- the immune disorder and/or inflammatory disorder is selected from the group of
   - a graft-versus-host disease (GvHD),
   - a transplant rejection, e.g., a rejection of a transplanted organ or tissue, in particular a host-versus-graft disease (HvGD),
   - autoimmune disorders, e.g.,
      - autoimmune intestinal or bowel disorders, e.g., Crohn's disease or ulcerative colitis,
      - autoimmune skin and/or ocular disorders, e.g., psoriasis, vitiligo, atopic dermatitis, uveitis, in particular autoimmune uveitis, for example refractory autoimmune uveitis,
      - autoimmune musculoskeletal disorders, e.g., arthritis, in particular rheumatoid arthritis (RA) or juvenile idiopathic arthritis (JIA), ankylosing spondylitis (Bekhterev's disease), autoimmune myositis, autoimmune polychondritis,
      - autoimmune connective tissue diseases (autoimmune collagenoses), e.g., Sjögren's syndrome (SjS, SS), lupus erythematosus, in particular systemic lupus erythematosus (SLE), systemic sclerosis, in particular systemic sclerosis-associated interstitial lung disease (SSc-ILD), autoimmune vasculitis,
      - neuroinflammation-associated neurodegenerative disorders, e.g., Alzheimer's disease (AD), Parkinson's disease (PD), amyotrophic lateral sclerosis (ALS), neurodegenerative disorders, multiple sclerosis (MS),
      - other autoimmune disorders, e.g., autoimmune hepatitis, type 1 diabetes mellitus, lupus nephritis, myasthenia gravis, Grave's disease, and
   - low-grade inflammation and chronic low-grade inflammation, disorders associated with low-grade inflammation and chronic low-grade inflammation, e.g., myalgic encephalomyelitis (ME), chronic fatigue syndrome (CFS), myalgic encephalomyelitis/chronic fatigue syndrome (ME/CFS), post-viral fatigue syndrome (PVFS), post-COVID syndrome (PCS, long COVID), adverse reactions after COVID-19 vaccination (Post Vac).

A second aspect of the invention relates to a composition comprising platelet derived extracellular vesicles for use in the prevention and/or treatment of an immune disorder and/or of an inflammatory disorder in a subject, wherein the composition is administered to the subject,
wherein
- the composition is administered to the subject in combination with at least one drug, wherein the at least one drug is selected from the group of immunosuppressive and/or anti-inflammatory drugs.

According to the second aspect of the invention, the immune disorder and/or inflammatory disorder may be any immune disorder and/or inflammatory disorder. Preferably, the immune disorder and/or inflammatory disorder may be accompanied by an excessive immune response. The immune disorder and/or inflammatory disorder may, for example, be selected from the group of
- a graft-versus-host disease (GvHD),
- a transplant rejection, e.g., a rejection of a transplanted organ or tissue, in particular a host-versus-graft disease (HvGD),
- autoimmune disorders, e.g.,
   - autoimmune intestinal or bowel disorders, e.g., Crohn's disease or ulcerative colitis,
   - autoimmune skin and/or ocular disorders, e.g., psoriasis, vitiligo, atopic dermatitis, uveitis, in particular autoimmune uveitis, for example refractory autoimmune uveitis,
   - autoimmune musculoskeletal disorders, e.g., arthritis, in particular rheumatoid arthritis (RA) or juvenile idiopathic arthritis (JIA), ankylosing spondylitis (Bekhterev's disease), autoimmune myositis, autoimmune polychondritis,
   - autoimmune connective tissue diseases (autoimmune collagenoses), e.g., Sjögren's syndrome (SjS, SS), lupus erythematosus, in particular systemic lupus erythematosus (SLE), systemic sclerosis, in particular systemic sclerosis-associated interstitial lung disease (SSc-ILD), autoimmune vasculitis,
   - neuroinflammation-associated neurodegenerative disorders, e.g., Alzheimer's disease (AD), Parkinson's disease (PD), amyotrophic lateral sclerosis (ALS), neurodegenerative disorders, multiple sclerosis (MS),
   - other autoimmune disorders, e.g., autoimmune hepatitis, type 1 diabetes mellitus, lupus nephritis, myasthenia gravis, Grave's disease, and
- low-grade inflammation and chronic low-grade inflammation, disorders associated with low-grade inflammation and chronic low-grade inflammation, e.g., myalgic encephalomyelitis (ME), chronic fatigue syndrome (CFS), myalgic encephalomyelitis/chronic fatigue syndrome (ME/CFS), post-viral fatigue syndrome (PVFS), post-COVID syndrome (PCS, long COVID), adverse reactions after COVID-19 vaccination (Post Vac).

A third aspect of the invention relates to a composition comprising platelet derived extracellular vesicles for use in the prevention and/or treatment of an immune disorder and/or of an inflammatory disorder in a subject, wherein the composition is administered to the subject,
wherein
- the immune disorder and/or inflammatory disorder is selected from the group of
   - a graft-versus-host disease (GvHD),
   - a transplant rejection, e.g., a rejection of a transplanted organ or tissue, in particular a host-versus-graft disease (HvGD),
   - autoimmune disorders, e.g.,
      - autoimmune intestinal or bowel disorders, e.g., Crohn's disease or ulcerative colitis,
      - autoimmune skin and/or ocular disorders, e.g., psoriasis, vitiligo, atopic dermatitis, uveitis, in particular autoimmune uveitis, for example refractory autoimmune uveitis,
      - autoimmune musculoskeletal disorders, e.g., arthritis, in particular rheumatoid arthritis (RA) or juvenile idiopathic arthritis (JIA), ankylosing spondylitis (Bekhterev's disease), autoimmune myositis, autoimmune polychondritis,
      - autoimmune connective tissue diseases (autoimmune collagenoses), e.g., Sjögren's syndrome (SjS, SS), lupus erythematosus, in particular systemic lupus erythematosus (SLE), systemic sclerosis, in particular systemic sclerosis-associated interstitial lung disease (SSc-ILD), autoimmune vasculitis,
      - neuroinflammation-associated neurodegenerative disorders, e.g., Alzheimer's disease (AD), Parkinson's disease (PD), amyotrophic lateral sclerosis (ALS), neurodegenerative disorders, multiple sclerosis (MS),
      - other autoimmune disorders, e.g., autoimmune hepatitis, type 1 diabetes mellitus, lupus nephritis, myasthenia gravis, Grave's disease, and
   - low-grade inflammation and chronic low-grade inflammation, disorders associated with low-grade inflammation and chronic low-grade inflammation, e.g., myalgic encephalomyelitis (ME), chronic fatigue syndrome (CFS), myalgic encephalomyelitis/chronic fatigue syndrome (ME/CFS), post-viral fatigue syndrome (PVFS), post-COVID syndrome (PCS, long COVID), adverse reactions after COVID-19 vaccination (Post Vac).

According to the third aspect of the invention, the composition may be administered to the subject as a sole medication, in combination with any further drug or drugs, or in combination with at least one drug, wherein the at least one drug is selected from the group of immunosuppressive and/or anti-inflammatory drugs. The at least one drug which is selected from the group of immunosuppressive and/or anti-inflammatory drugs, may preferably be selected from the groups of immunosuppressive and/or anti-inflammatory drugs described herein.

Preferably, in connection with the "composition comprising platelet derived extracellular vesicles for use" as specified herein, the use may, for example, be either a use in the prevention and/or treatment of an immune disorder and/or of an inflammatory disorder in a subject, wherein the composition is administered to the subject, wherein the composition is administered to the subject in combination with at least one drug, wherein the at least one drug is selected from the group of immunosuppressive and/or anti-inflammatory drugs [second aspect of the invention as mentioned herein]; or a use in the prevention and/or treatment of an immune disorder and/or of an inflammatory disorder in a subject, wherein the composition is administered to the subject, wherein
- the immune disorder and/or inflammatory disorder is selected from the group of
   - a graft-versus-host disease (GvHD),
   - a transplant rejection, e.g., a rejection of a transplanted organ or tissue, in particular a host-versus-graft disease (HvGD),
   - autoimmune disorders, e.g.,
      - autoimmune intestinal or bowel disorders, e.g., Crohn's disease or ulcerative colitis,
      - autoimmune skin and/or ocular disorders, e.g., psoriasis, vitiligo, atopic dermatitis, uveitis, in particular autoimmune uveitis, for example refractory autoimmune uveitis,
      - autoimmune musculoskeletal disorders, e.g., arthritis, in particular rheumatoid arthritis (RA) or juvenile idiopathic arthritis (JIA), ankylosing spondylitis (Bekhterev's disease), autoimmune myositis, autoimmune polychondritis,
      - autoimmune connective tissue diseases (autoimmune collagenoses), e.g., Sjögren's syndrome (SjS, SS), lupus erythematosus, in particular systemic lupus erythematosus (SLE), systemic sclerosis, in particular systemic sclerosis-associated interstitial lung disease (SSc-ILD), autoimmune vasculitis,
      - neuroinflammation-associated neurodegenerative disorders, e.g., Alzheimer's disease (AD), Parkinson's disease (PD), amyotrophic lateral sclerosis (ALS), neurodegenerative disorders, multiple sclerosis (MS),
      - other autoimmune disorders, e.g., autoimmune hepatitis, type 1 diabetes mellitus, lupus nephritis, myasthenia gravis, Grave's disease, and
   - low-grade inflammation and chronic low-grade inflammation, disorders associated with low-grade inflammation and chronic low-grade inflammation, e.g., myalgic encephalomyelitis (ME), chronic fatigue syndrome (CFS), myalgic encephalomyelitis/chronic fatigue syndrome (ME/CFS), post-viral fatigue syndrome (PVFS), post-COVID syndrome (PCS, long COVID), adverse reactions after COVID-19 vaccination (Post Vac) [third aspect of the invention as mentioned herein];
or a combination of both, the second and the third aspect [first aspect of the invention as mentioned herein].

The invention further relates to a composition, e.g., in the form of a combination drug composition, comprising platelet derived extracellular vesicles and at least one drug, wherein the at least one drug is selected from the group of immunosuppressive and/or anti-inflammatory drugs. This may be considered as a fourth aspect of the invention. This composition may preferably comprise platelet derived extracellular vesicles and at least two drugs, wherein at least one drug of the at least two drugs may preferably be selected from the group of immunosuppressive and/or anti-inflammatory drugs.

The invention further relates to a composition, e.g., in the form of a combination drug composition, comprising platelet derived extracellular vesicles and at least one drug, wherein the at least one drug is selected from the group of immunosuppressive and/or anti-inflammatory drugs, for use as a medicament. This may be considered as a fifth aspect of the invention. This composition may preferably comprise platelet derived extracellular vesicles and at least two drugs, wherein at least one drug of the at least two drugs may preferably be selected from the group of immunosuppressive and/or anti-inflammatory drugs.

The invention further relates to a method for the prevention and/or treatment of an immune disorder and/or of an inflammatory disorder, comprising administering to said patient an effective amount of a composition comprising platelet derived extracellular vesicles. This may be considered as a sixth aspect of the invention. The composition may be any of the compositions described herein. The immune disorder and/or inflammatory disorder may be any of the immune disorders and/or inflammatory disorders described herein.

Of course, any information provided herein relating to a composition, e.g., to "a composition", to "the composition", to "a composition according to the invention", or "the composition according to the invention", may equally relate to the first, second, third, fourth, fifth or sixth aspect of the invention.

If not indicated otherwise, terms in round brackets are abbreviations or synonyms of preceding terms or expressions. The term "GvHD" is an abbreviation for "graft-versus-host disease". The term "aGvHD" is an abbreviation for "acute graft-versus-host disease". The term "cGvHD" is an abbreviation for "chronic graft-versus-host disease". The term "HvGD" is an abbreviation for "host-versus-graft disease". The term "RA" is an abbreviation for "rheumatoid arthritis". The term "JIA" is an abbreviation for "juvenile idiopathic arthritis". The term "Bekhterev's disease" is a synonym for "ankylosing spondylitis". The term "autoimmune collagenoses" is a synonym for "autoimmune connective tissue diseases". The terms "SjS" and "SS" are abbreviations for "Sjögren's syndrome". The term "SLE" is an abbreviation for "systemic lupus erythematosus". The term "SSc-ILD" is an abbreviation for "systemic sclerosis-associated interstitial lung disease". The term "AD" is an abbreviation for "Alzheimer's disease". The term "PD" is an abbreviation for "Parkinson's disease". The term "ALS" is an abbreviation for "amyotrophic lateral sclerosis". The term "MS" is an abbreviation for "multiple sclerosis". The term "ME" is an abbreviation for "myalgic encephalomyelitis". The term "CFS" is an abbreviation for "chronic fatigue syndrome". The term "ME/CFS" is an abbreviation for "myalgic encephalomyelitis/chronic fatigue syndrome". The term "PVFS" is an abbreviation for "post-viral fatigue syndrome". The term "PCS" is an abbreviation for "post-COVID syndrome". The term "long COVID" is a synonym for "post-COVID syndrome". The term "Post Vac" is a synonym for "adverse reactions after COVID-19 vaccination".

The term "drug" as used herein, preferably includes a pharmacologically active ingredient, pharmaceutically acceptable salts, and prodrugs thereof. A prodrug is a pharmacologically inactive or less active precursor of a drug that, after intake, is metabolized into a pharmacologically active ingredient. Instead of administering a drug directly, a corresponding prodrug can be used, for example, to improve how the drug is absorbed, distributed, metabolized, and excreted.

The term "group of immunosuppressive and/or anti-inflammatory drugs" as used herein, may in particular refer to or include the generally accepted and established but heterogeneous class of immunosuppressants.

The subject to which the composition can be administered is preferably a mammal, most preferably a human. The subject may be an adult, a child, or an infant.

The term "low-grade inflammation" is a synonym for "silent inflammation". A "low-grade inflammation" may, for example, be a chronic response to diseases, injuries, foreign and/or invaders, that produces a steady, low level of inflammation. The low level of inflammation may be present throughout the body.

Humans have the ability to produce inflammation as a way to deal with injury and disease. Sadly, this protective system may be turned against ourselves in a unique modern environment.

Low-grade inflammation may, for example, be defined by increased CRP levels. CRP (C-reactive protein) is one of the main inflammation biomarkers present in the body. The higher a CRP level, the more inflammation is present throughout a body. Low-grade inflammation can in particular be accompanied by CRP levels in a range from 3 mg to 10 mg per liter of blood.

The invention is based on several surprising discoveries.

It was found that several combinations of platelet derived extracellular vesicles with various already existing approved immunosuppressive and/or anti-inflammatory drugs provide unexpected beneficial immunosuppressive and/or anti-inflammatory effects.

Super-additive synergism was found regarding the inhibition of T cell proliferation and/or T cell activation.

It was further found that platelet derived extracellular vesicles exert unexpected cell-protective effects.

The invention thus offers promising for treatment, in particular for subjects suffering from immune and/or inflammatory disorders and for a prevention of immune and/or inflammatory disorders in subjects at risk of suffering from any such disorder in the future.

Preferably, the composition is a pharmaceutical composition.

According to the invention, the composition comprises platelet derived extracellular vesicles.

Extracellular vesicles commonly are known to transfer information between cells, organs and even between organisms, and have been detected in various body fluids, such as blood, urine, cerebrospinal liquid, breast milk and saliva. Exosomes and microvesicles comprise the most prominently described classes of extracellular vesicles. They are surrounded by a phospholipid membrane and may typically carry and/or contain cell-type-specific combinations of proteins, including enzymes, growth factors, receptors and cytokines as well as lipids, coding and non-coding RNAs, mRNA, microRNA (miRNA), or even small amounts of DNA, and metabolites. Exosomes are defined as 70-170 nm sized (+/- 20 nm, this varies depending on literature and technique of analysis) derivatives of the endosomal compartment. With average sizes of 100-1000 nm, microvesicles represent a class of larger extracellular vesicles that are formed by the outward budding of the plasma membrane. In the present application the term "extracellular vesicle" may comprise all of the above mentioned vesicles.

The term "platelet derived extracellular vesicles" herein relates in particular to vesicles that originate from platelets. These vesicles include vesicles that are secreted from platelets in a biological process into a surrounding medium and/or vesicles obtained by lysis and/or disruption of platelets. Herein, "EV" can, for example, be an abbreviation for "platelet derived extracellular vesicle".

The platelet derived extracellular vesicles are preferably platelet lysate derived extracellular vesicles. The platelet derived extracellular vesicles can preferably be derived from a platelet lysate.

The platelet derived extracellular vesicles are preferably *human* platelet derived extracellular vesicles. The platelet derived extracellular vesicles can preferably be derived from *human* platelet.

Preferably, the platelet derived extracellular vesicles are *human* platelet lysate derived extracellular vesicles. The platelet derived extracellular vesicles can preferably be derived from a *human* platelet lysate (hPL). This can, for example, be done as described in EP 3 468 568 B1.

Generally, the *human* platelet lysate may originate from any thinkable *human* blood sample comprising platelets. The *human* platelet lysate may originate from platelet concentrates such as, e.g., platelet rich plasma (PRP). PRP is a concentrate of platelets in plasma-derived from a patient's whole blood, centrifuged to remove red-blood cells and other unwanted components. It has a greater concentration of growth factors than whole blood and has been used as a tissue injection in a variety of disciplines, including dentistry, orthopedic surgery, and sports-medicine. The platelet-concentrates might, e.g., originate from buffy-coat extracted platelet concentrates or from platelet apheresis.

The platelet derived extracellular vesicles can, for example, originate from a single platelet concentrate or from more platelet concentrates.

A platelet concentrate may contain platelet of a single donor only. This platelet concentrate can, for example, be a platelet concentrate originating from apheresis, e.g., an apheresis-platelet concentrate. The preparation of apheresis-platelet concentrates is part of the common knowledge and also described in EP 3 468 568 B1.

A platelet concentrate may contain platelet of multiple donors, e.g., of three to five donors. This platelet concentrate can, for example, be a platelet concentrate originating from buffy-coats, e.g., a buffy coat-platelet concentrate. The preparation of buffy coat-platelet concentrates is part of the common knowledge and also described in EP 3 468 568 B1.

The platelet derived extracellular vesicles can, for example, originate from at least 10, preferably form at least 15, in particular from at least 20, e.g., from at least 40 donors.

The platelet derived extracellular vesicles can, for example, originate from at most 500, preferably form at most 350, in particular from at most 250, e.g., from at most 200 donors.

The platelet derived extracellular vesicles can, for example, originate from 10 to 500 donors, preferably from 15 to 350 donors, in particular from 20 to 250 donors, e.g., from 40 to 200 donors.

Platelet derived extracellular vesicles which originate from a specific number of donors can be obtained, for example, by pooling corresponding numbers of platelet concentrates to form a pooling product, lysing platelets comprised by the pooling product and enriching or purifying platelet derived extracellular vesicles from the platelet lysate.

All or part of the platelet derived extracellular vesicles can, preferably, originate from one or multiple platelet concentrate(s) that was or were stored at a temperature in a range from 18 °C to 26 °C, preferably in a range from 20 °C to 24 °C, for at least 2 days, in particular, for 2 to 10 days, e.g., for 4 to 7 days.

All or part of the platelet derived extracellular vesicles can, for example, originate from one or multiple platelet concentrate(s) that was or were stored at a temperature in a range from 20 °C to 24 °C, for 2 to 10 days, e.g., for 4 to 7 days.

All or part of the platelet derived extracellular vesicles can, for example, originate from one or multiple platelet concentrate(s) that was or were stored at a temperature in a range from 18 °C to 26 °C, preferably in a range from 20 °C to 24 °C until just before expiry, until expiry or until just after expiry. Storage until just before expiry is most preferred.

A platelet concentrate can be considered as expired as soon as it does no longer fulfill at least one of the criteria required for safe administration to humans.

The term "until just before expiry" may refer to any time at which, under the given storage conditions, at least 40 %, preferably at least 60 %, in particular at least 80 %, e.g., at least 95 %, of the time until expiry has passed, excluding the time of expiry and excluding any time after expiry.

The term "until expiry" may refer to any time at which, under the given storage conditions, the time until expiry +/-1 % has passed.

The term "until just after expiry" may, for example, refer to any time at which, under the given storage conditions, at most 150 %, in particular at most 130 %, e.g., at most 110 %, of the time until expiry has passed, excluding the time of expiry and excluding any time before expiry.

The term "until just before expiry" may refer to any time at which at least 48 hours, preferably at least 72 hours, in particular at least 96 hours, e.g., at least 114 hours, of the time until expiry has passed, excluding the time of expiry and excluding any time after expiry.

The term "until expiry" may refer to any time at which, under the given storage conditions, the time until expiry +/- 1 hour, has passed.

The term "until just after expiry" may, for example, refer to any time at which at most 180 hours, in particular at most 156 hours, e.g., at most 132 hours of the time until expiry has passed, excluding the time of expiry and excluding any time before expiry.

As explained in EP 3 468 568 B1, platelet derived extracellular vesicles can, for example, be obtained in the form of a fraction that is enriched for *human* platelet lysate derived extracellular vesicles.

The use as specified herein, i.e. the "use in the prevention and/or treatment of an immune disorder and/or of an inflammatory disorder in a subject, wherein the composition is administered to the subject" may be autologous. The platelet derived extracellular vesicles may be derived from and/or originate from platelet of the same subject, e.g., human.

The use as specified herein, i.e. the "use in the prevention and/or treatment of an immune disorder and/or of an inflammatory disorder in a subject, wherein the composition is administered to the subject" may be allogeneic. The platelet derived extracellular vesicles may be derived from and/or originate from platelet of one or more individuals of the same species, e.g. from one or more humans other than the subject.

The use as specified herein, i.e. the "use in the prevention and/or treatment of an immune disorder and/or of an inflammatory disorder in a subject, wherein the composition is administered to the subject" may be xenogeneic. The platelet derived extracellular vesicles may be derived from and/or originate from platelet of one or more individuals of another species, e.g., from one or more mammals of another species than the subject.

Most preferably, the use as specified herein, i.e. the "use in the prevention and/or treatment of an immune disorder and/or of an inflammatory disorder in a subject, wherein the composition is administered to the subject" is allogeneic.

The platelet derived extracellular vesicles comprised by the composition, e.g., the *human* platelet lysate derived extracellular vesicles comprised by the composition, may have a size from 30 nm to 1000 nm, in particular from 40 nm to 400 nm, further preferred from 40 nm to 300 nm, most preferred from 50 nm to 200 nm, e.g., from 60 to 190 nm.

Preferably, at least 90 % of all extracellular vesicles comprised by the composition may have a size from 30 nm to 1000 nm, in particular from 40 nm to 400 nm, further preferred from 40 nm to 300 nm, most preferred from 50 nm to 200 nm, e.g., from 60 to 190 nm.

Mean particle size and size distributions can be determined using Nanoparticle Tracking Analysis (NTA), for example, Nanosight or Zetaview, e.g., as described in detail in EP 3 468 568 B1, in particular in EP 3 468 568 B1, paragraph 131.

Preferably, platelet derived extracellular vesicles comprised by the composition are not contained in platelets.

Preferably, the composition does not contain platelets.

Preferably, the composition may be cell-free. Cell-free in the context of the present invention means that the composition is substantially free of living intact cells and cell-fragments.

The extracellular vesicles comprised by the composition can preferably be positive for at least one of the exosome markers consisting of the group: CD9, CD63, Hsp70, TSG101, and Syntenin. Preferably the extracellular vesicles are positive for 2, 3, 4, or 5 of these exosome markers.

Being positive according to the present invention means that the background medium fluorescence intensity of one of the above mentioned positive surface markers when carrying out a multiplex bead-based platform analysis according to the method described in Koliha, N. et al. (2016, Journal of extracellular vesicles, 5, 29975, is higher than a comparison example with extracellular vesicles deriving from NK cells.

Alternatively, other techniques for specific protein detection of above mentioned markers can be applied, such as Western Blot Analysis.

The extracellular vesicles comprised by the composition can preferably be negative for at least one of the cellular exosome markers consisting of the group CD 81, CD3, CD4, CD19, CD20, CD2, CD8, CD11a and CD25. Preferably the extracellular vesicles are negative for 2, 3, 4, 5, 6, 7, 8 or 9 of these cellular exosome markers.

To prove for the quality of extracellular vesicles comprised by the composition and/or for a fraction of extracellular vesicles to be used for preparing the composition, several general characterization criteria on the biological and biophysical properties of contained EVs should be fulfilled. State of the art for such characterization is the criteria and standards recommended by the International Society for Extracellular Vesicles (ISEV). Based on the latest scientific knowledge in the EV-field these criteria should be used to attribute any specific biological cargo or function to EVs.

Basic quality criteria/standards:
1. Determination of protein content [mg/ml] by standard protein quantification methods using BCA-assays, similar assays like the Bradford-Assay, or instruments based on spectrometry (like the "NanoDrop").
2. Determination of mean particle size [nm] and size distribution [curves] using the Nanoparticle Tracking Analysis (NTA) platform, such as Nanosight and Zetaview, or Image-Stream Flow Cytometry, such as "Amnis" that allows analysis of EVs at a single particle level.
3. Semiquantitative detection of typical EV-marker proteins including EV- or exosomal proteins (SDS-PAGE, Western Blot, detection with specific antibodies, detection of the signal). In general, the EV content is highly dependent on the cellular origin, on pre-conditioning of producing cell-lines and on the preparation method. However, EVs such as exosomes present a common set of generally expected markers that can be used for their characterization. Most commonly used markers are tetraspanins (CD9, CD63, CD81), endosome-derived or membrane-binding proteins (TSG101, annexins, Rabs, Syntenin, flotillines), and chaperone proteins (HSC70, HSP90). In case of platelet derived extracellular vesicles, characteristically they are lacking CD81.
4. Determination of purity by semiquantitative comparison of cell lysates and of fractions of platelet lysate derived extracellular vesicles. Fractions of platelet lysate derived extracellular vesicles should not contain cellular residues like proteins from the endoplasmic reticulum (e.g. Grp94, Calnexin), Golgi apparatus (e.g. GM130), or mitochondriae (e.g. prohibitin, cytochrome C). Such markers can be used as negative markers. Additionally nucleic proteins (histones, argonaute/RISC complex) could be used as examples for negative controls.

For 3 + 4: Analytic approaches for the detection of typical EV-markers can include Western blot (WB), (high resolution-) flow cytometry, or global proteomic analysis using mass spectrometry techniques. Additional characterization of fractions of platelet lysate derived extracellular vesicles is based on the following methods:

### Molecular profiling

Analytic approaches for the protein-profiling of platelet derived extracellular vesicles that include Western blots (WB), (high-resolution-) flow-cytometry or global proteomic analysis using mass-spectrometry are also state of the art for characterization of other EV-markers, such as immunological markers, signaling markers, cytokines and other associated bioactive protein contents.

For profiling of RNA of platelet derived extracellular vesicles, microarray technology can be applied. Microarrays are a well-established technique for analyzing the expression of known fragments of nucleic acids using slide or chip-based media. Microarrays are available for screening mRNA, miRNA and long non-coding RNA (IncRNA) species.

Lipids and lipid-raft-associated proteins in vesicular membranes provide extracellular vesicles with stability and structural integrity. Compared to their cells of origin platelet derived extracellular vesicles should present a similar lipid composition.

The platelet derived extracellular vesicles may be enriched for phosphatidylserine, unsaturated phosphatidylethanolamine, unsaturated phosphatidylcholine, sphingomyelin, ganglioside and cholesterol. For identification of the lipid composition and ratio, mass-spectrometry, flow cytometry, or other conventional assays can be used.

According to the present invention the platelet derived extracellular vesicles may comprise polynucleotides and/or oligonucleotides, preferably RNA and/or DNA, e.g., mRNA, microRNA (miRNA) and/or DNA.

Most preferably, the platelet derived extracellular vesicles and/or the composition may be DNA-free.

According to the present invention the extracellular vesicles may comprise proteins, e.g., transcription factors, cytokines and/or growth factors.

The composition and/or the extracellular vesicles may comprise at least one growth factor, particularly one or more of, preferably all of PDGF, VEGF, FGF, EGF, TGF, e.g., TGF-β, and/or CTGF. Preferably, the composition may comprise 2, 3, 4, 5, or 6 of these growth factors. Platelet-derived growth factors (PDGFs) promote cell growth and generation, repair of blood vessels and collagen-production. Vascular endothelial growth factors (VEGFs) promote growth and generation of vascular endothelial cells. Fibroblast growth factors (FGFs) promote tissue repair, cell growth, collagen-production and hyaluronic acid production. Epithelial growth factor (EGF) promotes epithelial cell growth, angiogenesis and wound-healing. Transforming growth factors (TGFs), especially TGF-β, promote growth and neogenesis of epithelial cells and wound-healing. Connective tissue growth factors (CTGFs) promote wound-repair. The composition of the invention therefore may promote the formation of new fibroblasts. These new fibroblasts start elastic and healthy, producing new collagen and less metalloproteases. The restoration of fibroblasts (the major cells in synthesizing, maintaining and providing the structural framework) results in healthier, restored skin. PDGF has also been shown to increase fibroblast motility, allowing fibroblasts to relocate to the site of administration. Natural growth factors (such as PDGF, VEGF, FGF, EGF, and TGF) found in the alpha-granules of platelets promote collagen and hyaluronic acid production, tissue repair, growth and regeneration of endothelial cells and epithelial cells, and new blood vessel formation (which restores oxygen and removes undesired molecules). All of these factors help to regenerate wrinkled and damaged extracellular matrix (ECM) back to its healthy state. Each of these growth factors plays a role within skin regeneration and restoration, both individually and additively in concert with each other. Treatments that stimulate the production of new, non-fragmented collagen will provide substantial improvement to the appearance and health of age.

The composition and/or extracellular vesicles may comprise cytokines, e.g., IL-8, IL-10, TGF-βI, and HLA-G, RANTES, Nap-2 and/or nucleic acids, such as, for example, microRNAs.

The composition according to the present invention might contain cytokines that possess antimicrobial properties. An inventive composition and/or extracellular vesicles might particularly comprise the cytokine RANTES or the cytokine NAP-2, e.g., elevated concentrations of at least one of the cytokines RANTES or NAP-2.

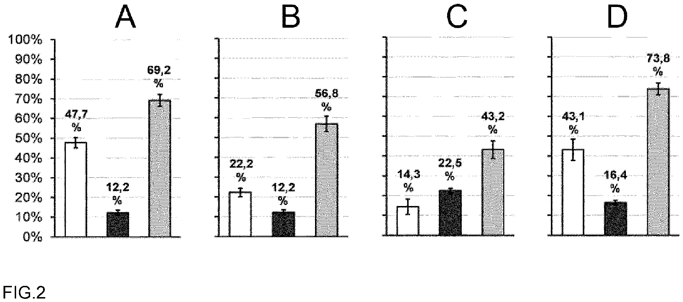

The cytokines RANTES and NAP-2 have been described for their antimicrobial properties e.g. in the article Mariani et al. (2015) (BMC Microbiology 15:149). The antimicrobial properties of the preparation according to the present invention can be measured according to the method underlying Fig. 2-Fig. 7 of Mariani *et al.* and serves as reference method.

According to the invention, the at least one drug which is selected from the group of immunosuppressive and/or anti-inflammatory drugs may preferably comprise at least one immunosuppressive and/or anti-inflammatory drug which is not prednisolone. The at least one drug which is selected from the group of immunosuppressive and/or anti-inflammatory drugs may, for example, comprise at least one immunosuppressive and/or anti-inflammatory drug which is not a corticosteroid.

According to the invention, the at least one drug which is selected from the group of immunosuppressive and/or anti-inflammatory drugs may preferably comprise at least one immunosuppressive and/or anti-inflammatory drug which is not infliximab. The at least one drug which is selected from the group of immunosuppressive and/or anti-inflammatory drugs may, for example, comprise at least one immunosuppressive and/or anti-inflammatory drug which is neither infliximab nor adalimumab nor etanercept. The at least one drug which is selected from the group of immunosuppressive and/or anti-inflammatory drugs may, for example, comprise at least one immunosuppressive and/or anti-inflammatory drug which is neither an anti-inflammatory antibody nor a TNF-alpha inhibitor.

According to the invention, the at least one drug which is selected from the group of immunosuppressive and/or anti-inflammatory drugs may preferably comprise at least one immunosuppressive and/or anti-inflammatory drug which is neither a corticosteroid nor an anti-inflammatory antibody nor a TNF-alpha inhibitor.

Such combination therapies can be particularly promising for many patients. A combined use of platelet derived extracellular vesicles with a range of well-known immunosuppressive and/or anti-inflammatory drugs resulted in a strong T cell inhibition, i. e., inhibition of T cell activation and/or T cell proliferation.

As shown in the examples, super-additive T cell inhibition was observed, for example, in combination with a broad range of subclasses of immunosuppressive and/or anti-inflammatory drugs, e.g. with calcineurin inhibitors, with an mTOR inhibitor, with inhibitors of nucleotide synthesis, with a kinase inhibitor and with an anti-inflammatory antibody. Super-additive inhibition of T cells thus appears to be essentially independent of the mechanism of action of the drug used in combination with platelet derived extracellular vesicles.

Surprisingly, the inhibition of T cells turned out to be super-additive with a broad range of different immunosuppressive and/or anti-inflammatory drugs. For prednisolone, the only corticosteroid tested, the observed effects were weaker than for other subclasses. For most of the subclasses tested, super-additive effects appeared to be stronger than for infliximab which is an anti-inflammatory antibody that is capable of inhibiting TNF-alpha.

According to the invention, the composition can preferably be administered to the subject in combination with at least two drugs, wherein the at least two drugs are selected from the group of immunosuppressive and/or anti-inflammatory drugs.

The at least two drugs which are selected from the group of immunosuppressive and/or anti-inflammatory drugs can preferably comprise at least one immunosuppressive and/or anti-inflammatory drug which is not prednisolone. The at least two drugs which are selected from the group of immunosuppressive and/or anti-inflammatory drugs may, for example, comprise at least one immunosuppressive and/or anti-inflammatory drug which is not a corticosteroid.

According to the invention, the at least two drugs which are selected from the group of immunosuppressive and/or anti-inflammatory drugs may preferably comprise at least one immunosuppressive and/or anti-inflammatory drug which is not infliximab. The at least two drugs which are selected from the group of immunosuppressive and/or anti-inflammatory drugs may, for example, comprise at least one immunosuppressive and/or anti-inflammatory drug which is neither infliximab nor adalimumab nor etanercept. The at least one drug which is selected from the group of immunosuppressive and/or anti-inflammatory drugs may, for example, comprise at least one immunosuppressive and/or anti-inflammatory drug which is neither an anti-inflammatory antibody nor a TNF-alpha inhibitor.

According to the invention, the at least two drugs which are selected from the group of immunosuppressive and/or anti-inflammatory drugs may preferably comprise at least one immunosuppressive and/or anti-inflammatory drug which is neither a corticosteroid nor an anti-inflammatory antibody nor a TNF-alpha inhibitor.

At least one of the at least one or two drugs can preferably be selected from the following drugs:
- calcineurin inhibitors, e.g., cyclosporine, aerosolized cyclosporine, voclosporin, tacrolimus, pimecrolimus,
- inhibitors of nucleotide synthesis and/or antimetabolites and/or nucleoside or nucleobase analogues, e.g., methotrexate, mycophenolate mofetil, mycophenolate sodium, mizoribine, azathioprine, leflunomide, teriflunomide, cladribine,
- kinase inhibitors, in particular JAK inhibitors, e.g., ruxolitinib, abrocitinib, oclacitinib, filgotinib, baricitinib, peficitinib, upadacitinib, fedratinib, delgocitinib tofacitinib, pacritinib, deucravacitinib, ritlecitinib, deucravacitinib,
- mTOR inhibitors, e.g., sirolimus, everolimus, temsirolimus, ridaforolimus, umirolimus, zotarolimus,
- alkylating agents, e.g., cyclophosphamide, chlorambucil,
- agents suppressing T cell co-stimulation, e.g., abatacept, belatacept,
- polyclonal and monoclonal antibodies against lymphocyte cell surface antigens, in particular against B lymphocyte and/or T lymphocyte cell surface antigens, e.g., alemtuzumab, natalizumab, ocrelizumab, ofatumumab
- deoxyspergualin,
- interferons, e.g., interferon beta-1a, interferon beta-1b, peginterferon beta-1a,
- fingolimod, myriocin, ozanimod, ponesimod, siponimod,
- glatiramer acetate, Copolymer 1, Cop-1,
- mitoxantrone, pixantrone, losoxantrone,
- stem cell derived extracellular vesicles,
- corticosteroids, e.g., clobetasol, halobetasol, hydrocortisone, triamcinolone, betamethasone, fluocinole, fluocinonide, prednisone, prednisolone, methylprednisolone,
- non-steroidal anti-inflammatory drugs, e.g., sulfasalazine, mesalamine, celecoxib, diclofenac, etodolac, fenprofen, flurbiprofen, ibuprofen, ketoprofen, meclofamate, meloxicam, nabumetone, naproxen, oxaprozin, piroxicam, rofecoxib, salicylates, sulindac, tolmetin,
- phosphodiesterase-4 inhibitors, e.g., rolipram,
- anti-inflammatory antibodies and/or TNF-alpha inhibitors, e.g., adalimumab, infliximab, etanercept,
- fumarates, e.g., dimethyl fumarate, diroximel fumarate.

(The above listed drugs are abbreviated as drugs of group A).

The above listed drugs are sorted, in essence, according to their usual classification into common and generally accepted subclasses of immunosuppressive and/or anti-inflammatory drugs.

At least one of the at least one or two drugs can preferably be selected from the following drugs:
- calcineurin inhibitors, e.g., cyclosporine, aerosolized cyclosporine, voclosporin, tacrolimus, pimecrolimus,
- inhibitors of nucleotide synthesis and/or antimetabolites and/or nucleoside or nucleobase analogues, e.g., methotrexate, mycophenolate mofetil, mycophenolate sodium, mizoribine, azathioprine, leflunomide, teriflunomide, cladribine,
- kinase inhibitors, in particular JAK inhibitors, e.g., ruxolitinib, abrocitinib, oclacitinib, filgotinib, baricitinib, peficitinib, upadacitinib, fedratinib, delgocitinib tofacitinib, pacritinib, deucravacitinib, ritlecitinib, deucravacitinib,
- mTOR inhibitors, e.g., sirolimus, everolimus, temsirolimus, ridaforolimus, umirolimus, zotarolimus,
- alkylating agents, e.g., cyclophosphamide, chlorambucil,
- agents suppressing T cell co-stimulation, e.g., abatacept, belatacept,
- polyclonal and monoclonal antibodies against lymphocyte cell surface antigens, in particular against B lymphocyte and/or T lymphocyte cell surface antigens, e.g., alemtuzumab, natalizumab, ocrelizumab, ofatumumab
- deoxyspergualin,
- interferons, e.g., interferon beta-1a, interferon beta-1b, peginterferon beta-1a,
- fingolimod, myriocin, ozanimod, ponesimod, siponimod,
- glatiramer acetate, Copolymer 1, Cop-1,
- mitoxantrone, pixantrone, losoxantrone,
- stem cell derived extracellular vesicles,
- corticosteroids excluding prednisolone, e.g., clobetasol, halobetasol, hydrocortisone, triamcinolone, betamethasone, fluocinole, fluocinonide, prednisone, methylprednisolone,
- non-steroidal anti-inflammatory drugs, e.g., sulfasalazine, mesalamine, celecoxib, diclofenac, etodolac, fenprofen, flurbiprofen, ibuprofen, ketoprofen, meclofamate, meloxicam, nabumetone, naproxen, oxaprozin, piroxicam, rofecoxib, salicylates, sulindac, tolmetin,
- phosphodiesterase-4 inhibitors, e.g., rolipram,
- anti-inflammatory antibodies and/or TNF-alpha inhibitors, e.g., adalimumab, infliximab, etanercept,
- fumarates, e.g., dimethyl fumarate, diroximel fumarate.

(The above listed drugs are abbreviated as drugs of group A-p). The drugs of group A-p include the drugs of group A, excluding prednisolone.

At least one of the at least one or two drugs can preferably be selected from the following drugs:
- calcineurin inhibitors, e.g., cyclosporine, aerosolized cyclosporine, voclosporin, tacrolimus, pimecrolimus,
- inhibitors of nucleotide synthesis and/or antimetabolites and/or nucleoside or nucleobase analogues, e.g., methotrexate, mycophenolate mofetil, mycophenolate sodium, mizoribine, azathioprine, leflunomide, teriflunomide, cladribine,
- kinase inhibitors, in particular JAK inhibitors, e.g., ruxolitinib, abrocitinib, oclacitinib, filgotinib, baricitinib, peficitinib, upadacitinib, fedratinib, delgocitinib tofacitinib, pacritinib, deucravacitinib, ritlecitinib, deucravacitinib,
- mTOR inhibitors, e.g., sirolimus, everolimus, temsirolimus, ridaforolimus, umirolimus, zotarolimus,
- alkylating agents, e.g., cyclophosphamide, chlorambucil,
- agents suppressing T cell co-stimulation, e.g., abatacept, belatacept,
- polyclonal and monoclonal antibodies against lymphocyte cell surface antigens, in particular against B lymphocyte and/or T lymphocyte cell surface antigens, e.g., alemtuzumab, natalizumab, ocrelizumab, ofatumumab
- deoxyspergualin,
- interferons, e.g., interferon beta-1a, interferon beta-1b, peginterferon beta-1a,
- fingolimod, myriocin, ozanimod, ponesimod, siponimod,
- glatiramer acetate, Copolymer 1, Cop-1,
- mitoxantrone, pixantrone, losoxantrone,
- stem cell derived extracellular vesicles,
- non-steroidal anti-inflammatory drugs, e.g., sulfasalazine, mesalamine, celecoxib, diclofenac, etodolac, fenprofen, flurbiprofen, ibuprofen, ketoprofen, meclofamate, meloxicam, nabumetone, naproxen, oxaprozin, piroxicam, rofecoxib, salicylates, sulindac, tolmetin,
- phosphodiesterase-4 inhibitors, e.g., rolipram,
- anti-inflammatory antibodies and/or TNF-alpha inhibitors, e.g., adalimumab, infliximab, etancercept,
- fumarates, e.g., dimethyl fumarate, diroximel fumarate.

(The above listed drugs are abbreviated as drugs of group A-c). The drugs of group A-c include the drugs of group A, excluding the corticosteroids.

At least one of the at least one or two drugs can preferably be selected from the following drugs:
- cyclosporine, aerosolized cyclosporine, voclosporin, tacrolimus, pimecrolimus,
- methotrexate, mycophenolate mofetil, mycophenolate sodium, mizoribine, azathioprine, leflunomide, teriflunomide, cladribine,
- ruxolitinib, abrocitinib, oclacitinib, filgotinib, baricitinib, peficitinib, upadacitinib, fedratinib, delgocitinib tofacitinib, pacritinib, deucravacitinib, ritlecitinib, deucravacitinib,
- sirolimus, everolimus, temsirolimus, ridaforolimus, umirolimus, zotarolimus,
- cyclophosphamide, chlorambucil,
- abatacept, belatacept,
- alemtuzumab, natalizumab, ocrelizumab, ofatumumab
- deoxyspergualin,
- interferon beta-1a, interferon beta-1b, peginterferon beta-1a,
- fingolimod, myriocin, ozanimod, ponesimod, siponimod,
- glatiramer acetate, Copolymer 1, Cop-1,
- mitoxantrone, pixantrone, losoxantrone,
- stem cell derived extracellular vesicles,
- clobetasol, halobetasol, hydrocortisone, triamcinolone, betamethasone, fluocinole, fluocinonide, prednisone, prednisolone, methylprednisolone,
- sulfasalazine, mesalamine, celecoxib, diclofenac, etodolac, fenprofen, flurbiprofen, ibuprofen, ketoprofen, meclofamate, meloxicam, nabumetone, naproxen, oxaprozin, piroxicam, rofecoxib, salicylates, sulindac, tolmetin,
- rolipram,
- adalimumab, infliximab, etancercept,
- dimethyl fumarate, diroximel fumarate.

(The above listed drugs are abbreviated as drugs of group A1). The drugs of group A1 include only specific drugs of group A.

At least one of the at least one or two drugs can preferably be selected from the following drugs:
- cyclosporine, aerosolized cyclosporine, voclosporin, tacrolimus, pimecrolimus,
- methotrexate, mycophenolate mofetil, mycophenolate sodium, mizoribine, azathioprine, leflunomide, teriflunomide, cladribine,
- ruxolitinib, abrocitinib, oclacitinib, filgotinib, baricitinib, peficitinib, upadacitinib, fedratinib, delgocitinib tofacitinib, pacritinib, deucravacitinib, ritlecitinib, deucravacitinib,
- sirolimus, everolimus, temsirolimus, ridaforolimus, umirolimus, zotarolimus,
- cyclophosphamide, chlorambucil,
- abatacept, belatacept,
- alemtuzumab, natalizumab, ocrelizumab, ofatumumab
- deoxyspergualin,
- interferon beta-1a, interferon beta-1b, peginterferon beta-1a,
- fingolimod, myriocin, ozanimod, ponesimod, siponimod,
- glatiramer acetate, Copolymer 1, Cop-1,
- mitoxantrone, pixantrone, losoxantrone,
- stem cell derived extracellular vesicles,
- clobetasol, halobetasol, hydrocortisone, triamcinolone, betamethasone, fluocinole, fluocinonide, prednisone, methylprednisolone,
- sulfasalazine, mesalamine, celecoxib, diclofenac, etodolac, fenprofen, flurbiprofen, ibuprofen, ketoprofen, meclofamate, meloxicam, nabumetone, naproxen, oxaprozin, piroxicam, rofecoxib, salicylates, sulindac, tolmetin,
- rolipram,
- adalimumab, infliximab, etanercept,
- dimethyl fumarate, diroximel fumarate.

(The above listed drugs are abbreviated as drugs of group A1-p). The drugs of group A1-p include the drugs of group A1, excluding prednisolone.

At least one of the at least one or two drugs can preferably be selected from the following drugs:
- cyclosporine, aerosolized cyclosporine, voclosporin, tacrolimus, pimecrolimus,
- methotrexate, mycophenolate mofetil, mycophenolate sodium, mizoribine, azathioprine, leflunomide, teriflunomide, cladribine,
- ruxolitinib, abrocitinib, oclacitinib, filgotinib, baricitinib, peficitinib, upadacitinib, fedratinib, delgocitinib tofacitinib, pacritinib, deucravacitinib, ritlecitinib, deucravacitinib,
- sirolimus, everolimus, temsirolimus, ridaforolimus, umirolimus, zotarolimus,
- cyclophosphamide, chlorambucil,
- abatacept, belatacept,
- alemtuzumab, natalizumab, ocrelizumab, ofatumumab
- deoxyspergualin,
- interferon beta-1a, interferon beta-1b, peginterferon beta-1a,
- fingolimod, myriocin, ozanimod, ponesimod, siponimod,
- glatiramer acetate, Copolymer 1, Cop-1,
- mitoxantrone, pixantrone, losoxantrone,
- stem cell derived extracellular vesicles,
- sulfasalazine, mesalamine, celecoxib, diclofenac, etodolac, fenprofen, flurbiprofen, ibuprofen, ketoprofen, meclofamate, meloxicam, nabumetone, naproxen, oxaprozin, piroxicam, rofecoxib, salicylates, sulindac, tolmetin,
- rolipram,
- adalimumab, infliximab, etanercept,
- dimethyl fumarate, diroximel fumarate.

(The above listed drugs are abbreviated as drugs of group A1-c). The drugs of group A1-c include the drugs of group A1, excluding the corticosteroids.

At least one of the at least one or two drugs can optionally be selected from: Alpha Lipoic Acid, Nasturtium/Horseradish, annual mugwort Artemisia, tree bark extract, frankincense, curcumin, linseed oil, African frankincense, frankincense, devil's claw, Cysteine protease, green tea extract, vitamin C, Cannabidiol, Coriander, Cistus rose, Coenzyme Q 10 (Ubiquinone), Cholesterol inhibitor, First milk preparation, Cordyceps extract (Vital mushrooms), American cranberry, Black pepper extract, Curcumin (Curcuma), Curcumin/Vitamin D, Flavonoids, isoflavones, Omega 3 fish oil, Eleutherococcus root, Bromelain/Papain/Rutin, St. John's wort, Rosehip Hedgehog spikenard, Stinging nettle, Micronutrients, Karazym (enzyme preparation), Uridine monophosphate, Vit. B12, 5-methyltetrahydrofolic acid, Korean red ginseng, Ginsenosides, Krill oil, L-Carnitine, Linseed oil, Lidocaine, Methyl-Sulfonyl-Methane, white peony root, yellow ginger/yellow root, soy isoflavones and red clover, Indian frankincense, Colostrum, Vitamin D3, Agaricus, procaine, lecithin, polyphenols, probiotic microorganisms, bee propolis, pine extract, grape seed extract, onion extract, reduced glutathione, medicinal mushrooms, Polyphenol phytoalexin, grape seed extract, quercetin, Petroleum, furfural, balsamic turpentine oil, Rizol Omega, Borax, Chloroquine, S-adenosylmethionine, milk thistle, frankincense ginger, Niacin, green tea extract, curcuma, resveratrol, Frankincense serrata, willow bark, ginger, Lavender oil, Lemon oil, Lemongrass oil, Thyme oil, Rosemary oil, Manuka oil. (These drugs are abbreviated as drugs of Group B)

The at least one of the at least one or two drugs may preferably be methotrexate.

Preferably, at least two of the at least two drugs can be selected from the drugs of group A, of group A-p, of group A-c, of group A1, of group A1-p, or of group A1-c.

Preferably, at least one of the at least two drugs can be selected from the drugs of group A and at least another one of the at least two drugs can be selected from the drugs of group A-p, of group A-c, of group A1-p, or of group A1-c.

Preferably, at least one of the at least two drugs can be selected from the drugs of group A1 and at least another one of the at least two drugs can be selected from the drugs of group A-p, of group A-c, of group A1-p, or of group A1-c.

Preferably, at least one of the at least two drugs is not prednisolone.

Preferably, at least one of the at least two drugs is not a corticosteroid.

The at least two of the at least two drugs may preferably include methotrexate.

At least one of the at least one or two drugs can preferably be selected from the following drugs:
- calcineurin inhibitors, e.g., cyclosporine, aerosolized cyclosporine, voclosporin, tacrolimus, pimecrolimus,
- inhibitors of nucleotide synthesis and/or antimetabolites and/or nucleoside or nucleobase analogues, e.g., methotrexate, mycophenolate mofetil, mycophenolate sodium, mizoribine, azathioprine, leflunomide, teriflunomide, cladribine,
- kinase inhibitors, in particular JAK inhibitors, e.g., ruxolitinib, abrocitinib, oclacitinib, filgotinib, baricitinib, peficitinib, upadacitinib, fedratinib, delgocitinib tofacitinib, pacritinib, deucravacitinib, ritlecitinib, deucravacitinib,
- mTOR inhibitors, e.g., sirolimus, everolimus, temsirolimus, ridaforolimus, umirolimus, zotarolimus,
- anti-inflammatory antibodies and/or TNF-alpha inhibitors, e.g., adalimumab, infliximab, etanercept.

(The above listed drugs are abbreviated as drugs of group C).

At least one of the at least one or two drugs can preferably be selected from the following drugs:
- cyclosporine, aerosolized cyclosporine, voclosporin, tacrolimus, pimecrolimus,
- methotrexate, mycophenolate mofetil, mycophenolate sodium, mizoribine, azathioprine, leflunomide, teriflunomide, cladribine,
- ruxolitinib, abrocitinib, oclacitinib, filgotinib, baricitinib, peficitinib, upadacitinib, fedratinib, delgocitinib tofacitinib, pacritinib, deucravacitinib, ritlecitinib, deucravacitinib,
- sirolimus, everolimus, temsirolimus, ridaforolimus, umirolimus, zotarolimus,
- anti-inflammatory antibodies and/or TNF-alpha inhibitors, e.g., adalimumab, infliximab, etanercept.

(The above listed drugs are abbreviated as drugs of group C1). The drugs of group C1 include only specific drugs of group C.

Preferably, at least one of the at least two drugs can be selected from the drugs of group A and at least another one of the at least two drugs can be selected from the drugs of group C or of group C1.

Preferably, at least one of the at least two drugs can be selected from the drugs of group A1 and at least another one of the at least two drugs can be selected from the drugs of group C or of group C1.

Preferably, at least two of the at least two drugs can be selected from the drugs of group C or of group C1.

At least two of the at least two drugs can preferably be selected from:
- calcineurin inhibitors, e.g., cyclosporine, aerosolized cyclosporine, voclosporin, tacrolimus, pimecrolimus,
- inhibitors of nucleotide synthesis and/or antimetabolites and/or nucleoside or nucleobase analogues, e.g., methotrexate, mycophenolate mofetil, mycophenolate sodium, mizoribine, azathioprine, leflunomide, teriflunomide, cladribine,
- kinase inhibitors, in particular JAK inhibitors, e.g., ruxolitinib, abrocitinib, oclacitinib, filgotinib, baricitinib, peficitinib, upadacitinib, fedratinib, delgocitinib tofacitinib, pacritinib, deucravacitinib, ritlecitinib, deucravacitinib,
- mTOR inhibitors, e.g., sirolimus, everolimus, temsirolimus, ridaforolimus, umirolimus, zotarolimus.

(The above listed drugs are abbreviated as drugs of group D).

At least two of the at least two drugs can preferably be selected from:
- cyclosporine, aerosolized cyclosporine, voclosporin, tacrolimus, pimecrolimus,
- methotrexate, mycophenolate mofetil, mycophenolate sodium, mizoribine, azathioprine, leflunomide, teriflunomide, cladribine,
- ruxolitinib, abrocitinib, oclacitinib, filgotinib, baricitinib, peficitinib, upadacitinib, fedratinib, delgocitinib tofacitinib, pacritinib, deucravacitinib, ritlecitinib, deucravacitinib,
- sirolimus, everolimus, temsirolimus, ridaforolimus, umirolimus, zotarolimus.

(The above listed drugs are abbreviated as drugs of group D1). The drugs of group D1 include only specific drugs of group D.

As specified herein, the composition can be administered to the subject in combination with at least one drug, wherein the at least one drug is selected from the group of immunosuppressive and/or anti-inflammatory drugs.

The composition can be administered to the subject in combination with at least one immunosuppressive and/or anti-inflammatory drug.

An administration of the composition to the subject in combination with at least one immunosuppressive and/or anti-inflammatory drug, as specified herein, may include all different forms and ways of administering the composition and the at least one drug to the subject.

An administration of the composition to the subject in combination with at least one immunosuppressive and/or anti-inflammatory drug, as specified herein, does not require an administration of the composition and any drug of one or more of the at least one drug at the same time.

An administration of the composition to the subject in combination with at least one drug, as specified herein, does not require an administration of the composition and any drug of one or more of the at least one drug as constituents of a combination drug composition.

The composition and at least one of the at least one drug or the composition and at least one or at least two of the at least two drugs may be administered to the subject sequentially, in either order, or simultaneously.

The composition and at least one of the at least one drug or the composition and at least one or at least two of the at least two drugs may be administered to the subject separately or as constituents of a combined pharmaceutical dosage form.

The subject can, for example, be
- a subject receiving at least one of the at least one or at least two drugs,
- a subject under treatment with at least one of the at least one or at least two drugs, and/or
- a subject having a significant, e.g., a therapeutically effective, level of at least one of the at least one or at least two drugs, in the subject's blood or other body fluid
when the composition comprising platelet derived extracellular vesicles is administered to the subject or included in the subject's treatment regimen.

The subject can, for example, be
- a subject receiving the composition comprising platelet derived extracellular vesicles, and/or
- a subject under treatment with the composition comprising platelet derived extracellular vesicles
when at least one of the at least one or at least two drugs is administered to the subject or included in the subject's treatment regimen.

The composition comprising platelet derived extracellular vesicles may comprise at least one drug, wherein the at least one drug is selected from the group of immunosuppressive and/or anti-inflammatory drugs. The composition may, for example, be a combination drug composition comprising platelet derived extracellular vesicles and at least one drug, wherein the at least one drug is selected from the group of immunosuppressive and/or anti-inflammatory drugs.

A combination drug is a single dosage form that comprises two or more active ingredients. Herein, a "combination drug composition" can, for example, be a single dosage form that comprises the platelet derived extracellular vesicles and the at least one drug, wherein the at least one drug is selected from the group of immunosuppressive and/or anti-inflammatory drugs.

The composition may, for example, be a fixed-dose combination comprising platelet derived extracellular vesicles and at least one drug, wherein the at least one drug is selected from the group of immunosuppressive and/or anti-inflammatory drugs.

Herein, the term "fixed-dose combination" may, preferably, refer to a form of a combination drug composition which is a mass-produced product having a predetermined combination of drugs and respective dosages. The term "fixed-dose combination" may exclude specific forms of combination drug compositions which are obtained from customized polypharmacy via compounding.

The composition may, for example, be an FDC comprising platelet derived extracellular vesicles and at least one drug, wherein the at least one drug is selected from the group of immunosuppressive and/or anti-inflammatory drugs. The term "FDC" may, preferably, be an abbreviation for the term "fixed-dose combination".

The combination drug composition and/or fixed-dose combination and/or FDC may, for example, comprise at least one first drug, wherein the at least one first drug is selected from the group of immunosuppressive and/or anti-inflammatory drugs. The combination drug composition and/or fixed-dose combination and/or FDC may optionally comprise at least one second drug.

The combination drug composition and/or fixed-dose combination and/or FDC may, for example, comprise at least one first drug, wherein the at least one first drug is selected from the group of immunosuppressive and/or anti-inflammatory drugs,
wherein
- the composition is administered to the subject in combination with at least one second drug, wherein the at least one second drug is also selected from the group of immunosuppressive and/or anti-inflammatory drugs, wherein the at least one second drug may preferably not be a constituent of the combination drug composition.

The first drug can, in particular, be selected from group A, A-p, A-c, A1, A1-p, A1-c, B, C, C1, D or D1. The first drug can, for example, be selected from group A, A-p, A-c, A1, A1-p, A1-c, C, C1, D or D1.

The second drug can, in particular, be selected from group A, A-p, A-c, A1, A1-p, A1-c, B, C, C1, D or D1. The second drug can, for example, be selected from group A, A-p, A-c, A1, A1-p, A1-c, C, C1, D or D1.

If the first drug is selected from group A, A-p, A-c, A1, A1-p, A1-c, B, C, C1, D or D1, the second drug can preferably be selected from group A, A-p, A-c, A1, A1-p, A1-c, C, C1, D or D1.

If the second drug is selected from group A, A-p, A-c, A1, A1-p, A1-c, B, C, C1, D or D1, the first drug can preferably be selected from group A, A-p, A-c, A1, A1-p, A1-c, C, C1, D or D1.

The combination drug composition and/or fixed-dose combination and/or FDC and at least one of the at least one second drug may be administered to the subject sequentially, in either order, or simultaneously.

The platelet derived extracellular vesicles may, for example, be the essential pharmaceutically active ingredient in the preparation.

Being the essential pharmaceutically active ingredient can mean that the composition preparation is substantially free of other pharmaceutically active ingredients except of the platelet derived extracellular vesicles. The composition can, for example, be a composition which is neither a combination drug composition, nor a fixed-dose combination.

Preferably, the immune disorder and/or inflammatory disorder can be accompanied by an excessive immune response.

Preferably, the immune disorder and/or inflammatory disorder can be accompanied by a systemic inflammation, e.g., acute or chronic systemic inflammation.

The excessive immune response and/or systemic inflammation, e.g. acute or chronic systemic inflammation, can, for example, be accompanied or indicated by an increased number of lymphocytes, preferably of T cells and/or B cells, e.g., of T cells, in the subject's blood.

The excessive immune response and/or systemic inflammation, e.g. acute or chronic systemic inflammation, can, for example, be accompanied or indicated by an increased activity of lymphocytes, preferably of T cells and/or B cells, e.g., of T cells, in the subject's blood. Lymphocyte activity can, for example, be estimated from CD25-expression.

The excessive immune response and/or systemic inflammation, e.g. acute or chronic systemic inflammation, can, for example, be an excessive immune response which is detectable or detected by cytokine profiling.

An excessive immune response and/or systemic inflammation, e.g. acute or chronic systemic inflammation, can, for example, be accompanied or indicated by an increased level of at least one proinflammatory cytokine, in particular by an increased level of TNF-α, IL-1β, IL-6, IFN-γ and/or IP-10, e.g., by an increased level of TNF-α, IL-6 and/or IFN-γ, in the subject's blood.

An excessive immune response and/or systemic inflammation, e.g. acute or chronic systemic inflammation, can, for example, be accompanied or indicated by a decreased level of at least one anti-inflammatory cytokine, in particular by a decreased level of IL-10 and/or TGF-β in the subject's blood.

The subject can, for example, be a subject showing at least one of the following characteristics (i) to (v) indicative for an immune disorder and/or inflammatory disorder, an excessive immune response, and/or systemic inflammation:
(i) an increased number of lymphocytes, preferably of T cells and/or B cells, e.g., of T cells, in the subject's blood,
(ii) an increased activity of lymphocytes, preferably of T cells and/or B cells, e.g., of T cells, in the subject's blood,
(iii) a cytokine profile indicative for an immune disorder and/or inflammatory disorder, an excessive immune response, and/or systemic inflammation, for example:
(iv) an increased level of at least one proinflammatory cytokine, in particular an increased level of TNF-α, IL-1β, IL-6, IFN-γ and/or IP-10, e.g., an increased level of TNF-α, IL-6 and/or IFN-γ, in the subject's blood,
(v) a decreased level of at least one anti-inflammatory cytokine, in particular by a decreased level of IL-10 and/or TGF-β in the subject's blood.

The immune disorder and/or inflammatory disorder can, for example, be a GvHD, e.g., aGvHD, a cGvHD, a graft-versus-host disease characterized by the simultaneous presence of acute and chronic graft-versus-host disease features, or a transfusion-associated graft-versus-host disease (TA-GvHD).

Preferably, the immune disorder and/or inflammatory disorder can be an aGvHD, a cGvHD or a GvHD characterized by the simultaneous presence of acute and chronic graft-versus-host disease features.

The graft-versus-host disease which is characterized by the simultaneous presence of acute and chronic graft-versus-host disease features may, for example, be an overlap chronic graft-versus-host (overlap cGvHD) disease or an aGvHD with appearance of aGvHD symptoms from the hundredth day after transplantation.

GvHD is generally associated with bone marrow transplants and stem cell transplants. GvHD is characterized by inflammation in different organs. It is assumed that GvHD is accompanied by leukocytes of the donor recognizing the recipient (i.e. the host) as foreign (non-self). The leukocytes present within the transplanted tissue can attack the recipient's body's cells, which may lead to GvHD.

GvHD can also occur after a blood transfusion, known as transfusion-associated graft-versus-host disease (TA-GvHD). The term "TA-GvHD" is an abbreviation for "transfusion-associated graft-versus-host disease". TA-GvHD can be observed, for example, if the blood products used have not been gamma irradiated or treated with an approved leukocyte reduction system.

With regard to GvHD, a distinction is made between two forms, acute GvHD (aGvHD) and chronic GvHD (cGvHD). Two mixed forms of GvHD occur in addition to aGvHD and cGvHD. One of these mixed forms is called overlap cGvHD and has characteristics of both forms. The other summarises forms of aGvHD whose common feature is the appearance of aGvHD symptoms from the hundredth day after transplantation (Filipovich, A.H., et al. National Institutes of Health consensus development project on criteria for clinical trials in chronic graft-versus-host disease: I. Diagnosis and staging working group report. Biol Blood Marrow Transplant 2005 Vol. 11 No. 12, pages 945 to 956).

A treatment of an immune disorder and/or of an inflammatory disorder in a subject may involve extracorporeal photopheresis (abbreviation: ECP) as an instrumental measure. ECP is a special type of blood washing, e.g., apheresis, in which certain blood cells are subjected to treatment with ultraviolet light (phototherapy) outside the subject's body. This therapeutic procedure can be used to destroy lymphocytes relatively gently. Excessive or misdirected immune function of white blood cells can be alleviated by this light therapy, which acts directly on the cells causing the disease.

According to the invention, the use in the prevention and/or treatment of an immune disorder and/or of an inflammatory disorder, e.g. of a GvHD, may be a use in a subject under extracorporeal photophoresis therapy. The subject can be considered as "a subject under extracorporeal photophoresis therapy" if the subject's treatment plan includes extracorporeal photophoresis therapy. Administration of the composition may occur at any time, e.g., before extracorporeal photophoresis, after extracorporeal photophoresis, and/or during extracorporeal photophoresis.

The composition may, for example, be administered to the subject under extracorporeal photophoresis therapy in combination with an inhibitor of nucleotide synthesis and/or an antimetabolite and/or a nucleoside or nucleobase analogue, preferably with mycophenolate mofetil.

According to the invention, the immune disorder and/or inflammatory disorder may preferably involve or may be accompanied by an inflammation-associated ocular and/or corneal disease, e.g., by an uveitis or an autoimmune uveitis.

The immune disorder and/or inflammatory disorder, preferably the graft-versus-host disease (GvHD), may involve or may be accompanied by an inflammation-associated ocular and/or corneal disease, e.g., a graft-versus-host disease (GvHD)-associated dry eye disease and/or an uveitis, e.g., an autoimmune uveitis and/or a keratoconjunctivitis sicca.

The immune disorder and/or inflammatory disorder, preferably the graft-versus-host disease (GvHD), may involve a sicca syndrome affecting an eye and/or a mucosa, e.g., keratoconjunctivitis sicca and/or dry mouth.

In particular but not only for treating or preventing ocular and/or corneal diseases, the composition can preferably be an ophthalmic composition.

The ophthalmic composition can, preferably, be selected from eye drops, an eye bath, a preparation for use on the eye, a semi-solid preparation for use on the eye, and an eye insert.

The ophthalmic composition can, for example, be eye drops.

Preferably, the ophthalmic composition, e.g., the eye drops, can be a combination drug composition and/or fixed-dose combination and/or FDC.

Most preferably, the ophthalmic composition, e.g. the eye drops, can be a combination drug composition and/or fixed-dose combination and/or FDC containing the platelet derived extracellular vesicles and the at least one drug which is selected from the group of immunosuppressive and/or anti-inflammatory drugs.

The ophthalmic composition, e.g. the eye drops, can, for example, be a combination drug composition and/or fixed-dose combination and/or FDC containing the platelet derived extracellular vesicles and a corticosteroid, e.g. clobetasol, halobetasol, hydrocortisone, triamcinolone, betamethasone, fluocinole, fluocinonide, prednisone, prednisolone, methylprednisolone. The corticosteroid may, for example, be selected from clobetasol, halobetasol, hydrocortisone, triamcinolone, betamethasone, fluocinole, fluocinonide, prednisone, and methylprednisolone.

The ophthalmic composition, e.g., eye drops, can preferably be an aqueous composition, wherein the platelet derived extracellular vesicles are dispersed in an aqueous carrier medium. The aqueous carrier medium may be an aqueous solution.

The ophthalmic composition, e.g., eye drops, may contain a further drug. Preferably, the further drug can be selected from dexpanthenol, hyaluronic acid, antihistamines, sympathomimetics, beta receptor blockers, parasympathomimetics, parasympatholytics, prostaglandins, antibiotics, antifungals, or topical anesthetics.

The ophthalmic composition may, for example, contain a preservative, e.g., cetrimide.

The ophthalmic composition may, for example, be preservative-free.

The ophthalmic composition may, for example, be an artificial tear composition.

Artificial tear compositions may, for example, be in the form of lubricating eye drops. Artificial tear compositions are often used to relieve dryness and irritation of the ocular surface.

The ophthalmic composition, e.g., the artificial tear composition, may, for example, contain a hydrophilic polymer. Preferably, the hydrophilic polymer is a cellulose based polymer, e.g., hydroxypropyl methylcellulose, carboxymethyl cellulose, or hydroxypropyl cellulose; a polymeric alcohol, e.g., polyvinyl alcohol; a polycarboxylic acid, e.g. a polyacrylic acid; or polyvinylpyrrolidone.

The invention further relates to an ophthalmic device, wherein the ophthalmic device comprises:
- a vessel, and
- a composition as defined herein, preferably an ophthalmic composition as defined herein, e.g., eye drops and/or an artificial tear composition,
wherein the composition is in the vessel.

The vessel can preferably comprise at least one compressible wall or capsule. The compressible wall or capsule can be compressed, e.g., between fingers of a user, in order to release at least a portion and/or one or more drops of the composition from the vessel.

The user can be the subject or a person supporting the subject.

The ophthalmic device can be a single use device. The ophthalmic composition comprised in the vessel of the single use device may, for example, be preservative-free.

The wall or capsule can contain a polymer material. The wall or capsule can be made from a polymer material. The polymer material can be any polymer material common for making single use ophthalmic devices.

After bone marrow or stem cell transplantation, 30-60% of transplanted patients develop aGvHD, 30-50% cGvHD. Some of the GvHD patients do not respond to any of the conventional therapies, which is why they have a mortality rate of about 15-30%. Depending on the severity of the disease, both forms of GvHD, i.e., aGvHD and cGvHD, are divided into severity classes. For aGvHD, there are a total of four classes I to IV; I=mild, II=moderate; III=severe, IV=life-threatening. For cGvHD there are three classes I to III; I=mild, II=moderate, and III=severe.

In aGvHD, the organ systems mainly affected are skin, intestine and liver.

The currently valid definition of the National Institute of Health (NIH) distinguishes between classic aGvHD which continues up to day 100 after transplantation, "late-onset" aGvHD occurring from day 100 after transplantation, persistent GvHD, and recurrent aGvHD with recurrence after a disease-free interval.

In order to determine the classification of aGvHD into the overall severity levels I-IV, the organ systems mainly affected (skin, intestine, liver) are first successively classified into four severity levels, 1 to 4, depending on the detectable extent of the damage. This results in the final classification I-IV; i.e., I=mild, II=moderate; III=severe, or IV=life-threatening. This classification of aGvHD into four degrees of severity is historically derived from the "Glucksberg-Seattle" criteria from 1974 (Glucksberg, H. et al. Clinical manifestations of graft-versus-host disease in human recipients of marrow from HL-A-matched sibling donors. Transplantation 1974, Vol. 18, No. 4, pages 295 to 304) and the "Consensus Conference on Acute GvHD Grading", which took place in 1994 (Przepiorka, D. et al. Consensus Conference on Acute GvHD Grading. Bone Marrow Transplant. 1994 Vol. 15 No. 6, page 825).

The GvHD can, for example, be an acute graft-versus-host disease (aGvHD). The subject may, in particular, have an aGvHD clinical grading of I, II, III or IV. The subject may, preferably, have an aGvHD clinical grading of II, III or IV. The subject may, most preferably, have an aGvHD clinical grading of III or IV. The subject may, for example, have an aGvHD clinical grading of IV. A clinical grading of I corresponds to the class I of the four classes I to IV of a GvHD. A clinical grading of II corresponds to the class II of the four classes I to IV of a GvHD. A clinical grading of III corresponds to the class III of the four classes I to IV of a GvHD. A clinical grading of IV corresponds to the class IV of the four classes I to IV of a GvHD.

The aGvHD may, in particular, be a classic aGvHD, a persistent aGvHD, a recurrent aGvHD, or a late-onset aGvHD.

The GvHD can, for example, be a cGvHD.

Chronic GvHD generally occurs at least 100 days after transplantation and can, in principle, affect all organs of the body. Symptoms can last for years or even decades.

Three forms of cGvHD are distinguished. "Progressive cGvHD", "quiescent cGvHD" and "de novo cGvHD". Progressive cGvHD develops directly after aGvHD. Quiescent cGvHD follows a symptom-free pause between the resolution of aGvHD and the onset of cGvHD. De novo cGvHD develops without the transplanted patient having aGvHD previously. The progressive onset of the disease, e.g., progressive cGvHD, is considered prognostically unfavorable (Arora, M. et al. Chronic graft-versus-host disease: a prospective cohort study. Biol Blood Marrow Transplant. 2003, Vol. 9, No. 1, 38-45; Kuzmina, Z. et al. Significantly worse survival of patients with NIH-defined chronic graft-versus-host disease and thrombocytopenia or progressive onset type: results of a prospective study. 2011; Leukemia, Vol. 26 No. 4, pages 746 to 756).

In order to classify cGvHD into one of the overall severity levels I to III, the organ systems mainly affected, e.g., skin, mouth, eyes, gastrointestinal tract, liver, lungs, joints and female genitalia, are first successively classified into three severity levels, 1 to 3, depending on the detectable extent of the damage. The assessment of the severity of the respective organ system is based on the resulting restrictions of activities in daily life (Filipovich, A.H., et al. National Institutes of Health consensus development project on criteria for clinical trials in chronic graft-versus-host disease: I. Diagnosis and staging working group report. Biol Blood Marrow Transplant 2005 Vol. 11 No. 12, pages 945 to 956). The overall picture of the number of organ systems affected in each case, as well as the severity of the organ manifestation, results in the final classification I, II, or III (I=mild, II=moderate, III=severe).

This classification of cGvHD into three overall severity levels is historically derived from the "Glucksberg-Seattle" criteria from 1974 (Glucksberg, H. et al. Clinical manifestations of graft-versus-host disease in human recipients of marrow from HL-A-matched sibling donors. Transplantation 1974, Vol. 18 No. 4, pages 295 to 304) and a revision from 2003 (Lee, S.J. et al. Chronic graft-versus-host disease. Biology of Blood and Marrow Transplantation, 2003 Vol. 9 Nr. 4, pages 215-233), according to which cGvHD was initially classified clinically into "limited" cGvHD and "extensive" cGvHD.

According to the invention, a global severity level of the cGvHD may be mild, moderate, or severe; most preferable moderate or severe; e.g., severe.

Preferably, a global severity level of the cGvHD may be mild, moderate, or severe; most preferable moderate or severe; e.g., severe.

Preferably, the cGvHD may be a classic chronic graft-versus-host disease, a progressive cGvHD, a quiescent cGvHD, or a de novo cGvHD.

According to the invention, the immune disorder and/or inflammatory disorder, e.g., the GvHD, may affect one or more of the following organs or tissues: skin, gut, intestine, liver, mucosa, mouth, eyes, lung, heart, and/or gastrointestinal system, e.g., skin, mucosa, mouth, eyes and/or lung. The use as defined herein may be a use in the prevention and/or treatment of an immune disorder and/or inflammatory disorder wherein the composition is administered to a skin, mucosa, mouth, eye and/or lung of the subject. The use as defined herein may be a use in the prevention and/or treatment of GvHD wherein the composition is administered to a skin, mucosa, mouth, eye and/or lung of the subject.

The composition, e.g., the combination drug composition and/or the fixed-dose combination and/or the FDC, may, for example, be suitable for
- intravenous administration,
- infusion,
- intraperitoneal injection,
- subcutaneous injection,
- intra bone injection,
- intracerebroventricular injection,
- intra muscular injection,
- intraocular injection,
- intra-articular injection or periarticular injection,
- peritendinous injection,
- intrathecal injection, or
- topical administration, e.g.,
   - topical administration to the skin,
   - topical administration to a mucous membrane,
   - ophthalmic administration, e.g. eye drops, or
   - inhalational topic administration;
   preferably suitable for
- intravenous administration,
- infusion,
- subcutaneous injection,
- intra muscular injection,
- intra-articular injection or periarticular injection,
- peritendinous injection,
- intrathecal injection, or
- topical administration, e.g.,
   - topical administration to the skin,
   - topical administration to a mucous membrane,
   - ophthalmic administration, e.g. eye drops,
   - inhalational topic administration,
   - intraoral, periodontal, or interdental application.

The composition, e.g., the combination drug composition and/or the fixed-dose combination and/or the FDC, may, for example, be a pharmaceutical composition for
- intravenous administration,
- infusion,
- intraperitoneal injection,
- subcutaneous injection,
- intra bone injection,
- intracerebroventricular injection,
- intra muscular injection,
- intraocular injection,
- intra-articular injection or periarticular injection,
- peritendinous injection,
- intrathecal injection, or
- topical administration, e.g.,
   - topical administration to the skin,
   - topical administration to a mucous membrane,
   - ophthalmic administration, e.g. eye drops, or
   - inhalational topic administration;
   preferably a pharmaceutical composition for
- intravenous administration,
- infusion,
- subcutaneous injection,
- intra muscular injection,
- intra-articular injection or periarticular injection,
- peritendinous injection,
- intrathecal injection, or
- topical administration, e.g.,
   - topical administration to the skin,
   - topical administration to a mucous membrane,
   - ophthalmic administration, e.g. eye drops,
   - inhalational topic administration,
   - intraoral, periodontal, or interdental application.

The composition, e.g., the combination drug composition and/or the fixed-dose combination and/or the FDC, may preferably be aqueous and/or comprise an aqueous carrier medium. All or part of the platelet derived extracellular vesicles can preferably be dispersed and/or embedded in the aqueous carrier medium.

The composition, e.g., the combination drug composition and/or the fixed-dose combination and/or the FDC, can preferably comprise at least 30 wt.-%, in particular at least 45 wt.-%, e.g. at least 60 wt.-% of water per total mass of the composition. The amount of water per total mass of the composition includes the water within vesicles.

An aqueous carrier medium and/or such elevated water content is preferred irrespective of the desired route of administration. This is because the stability and/ or activity of the platelet derived extracellular vesicles may be affected in certain non-aqueous media, e.g. in lipophilic media or non-polar solvents which may even dissolve the lipids of the vesicles and thereby destroy the vesicles.

The composition which is suitable for intravenous administration and/or the pharmaceutical composition for intravenous administration may preferably comprise water, at least part of the platelet derived extracellular vesicles dispersed in the water, optionally at least one drug which is selected from the group of immunosuppressive and/or anti-inflammatory drugs, and ions dissolved in the water, e.g., cations which are preferably selected from Na⁺, K⁺, Mg⁺, Fe²⁺, Fe³⁺, and/or Ca²⁺, and anions which may, in particular, include CI⁻, in amounts that are acceptable for intravenous administration. The composition which is suitable for intravenous administration and/or the pharmaceutical composition for intravenous administration may be a crystalloid or a colloid.

The composition which is suitable for infusion and/or the pharmaceutical composition for infusion may preferably comprise water, at least part of the platelet derived extracellular vesicles dispersed in the water, optionally at least one drug which is selected from the group of immunosuppressive and/or anti-inflammatory drugs, and ions dissolved in the water, e.g., cations which are preferably selected from Na⁺, K⁺, Mg⁺, Fe²⁺, Fe³⁺, and/or Ca²⁺, and anions which may, in particular, include CI⁻, in amounts that are acceptable for infusion therapy. The composition which is suitable for infusion and/or the pharmaceutical composition for infusion may be a crystalloid or a colloid.

The composition which is suitable for topical administration and/or the pharmaceutical composition for topical administration may be a topical pharmaceutical composition. Preferably, this composition, e.g., the topical pharmaceutical composition, may be a cream, a foam, a gel, a lotion, an ointment, an ophthalmic composition, or an inhalable composition.

The aqueous carrier medium of the composition which is suitable for topical administration and/or of the pharmaceutical composition for topical administration may, for example, comprise a hydrogel composition.

The aqueous carrier medium of the composition which is suitable for topical administration and/or of the pharmaceutical composition for topical administration may, for example, be embedded in or absorbed by a water-containing hydrogel composition.

The composition according to the invention, e.g., the combination drug composition and/or the fixed-dose combination and/or the FDC, may, for example, be a water-containing hydrogel composition.

Water-containing hydrogel compositions are part of the general knowledge in the technical filed of topical pharmaceutical compositions. Specific examples of water-containing hydrogel compositions are described in WO 2018/115453 A1. Branching and/or length of polymer chains of a polymer comprised by water-containing hydrogel composition can be optimized such that the vesicles can be released form the water-containing hydrogel composition.

The composition which is suitable for ophthalmic administration and/or the pharmaceutical composition for ophthalmic administration may be an ophthalmic composition as defined herein.

The composition which is suitable for intra-articular injection or peri-articular injection may preferably contain a pharmaceutically acceptable hygroscopic agent, e.g., hyaluronic acid, or a pharmaceutically acceptable salt thereof.

The composition which is suitable for peritendinous injection may preferably contain a pharmaceutically acceptable hygroscopic agent, e.g., hyaluronic acid or a pharmaceutically acceptable salt thereof.

The composition which is suitable for intrathecal injection may preferably contain an antispasmodic agent.

The composition which is suitable for intraoral, periodontal, or interdental application may preferably contain an analgesic agent and/or an antibiotic agent.

The immune disorder and/or inflammatory disorder, e.g., the GvHD, may preferably affect one or more of the following organs or tissues: skin, mucosa, mouth, eyes, and/or lung. Preferably, the administration to the subject comprises an administration to an affected skin, mucosa, mouth, eye, and/or lung.

Preferably, the composition comprising platelet derived extracellular vesicles can be a composition which is suitable for topical administration and/or the pharmaceutical composition for topical administration, for example, a composition which is suitable for topical administration and/or the pharmaceutical composition for topical administration, as defined herein. Preferably, the use in the prevention and/or treatment of an immune disorder and/or of an inflammatory disorder in a subject comprises a topical application of the composition to an organ or tissue affected by the immune disorder and/or inflammatory disorder. Preferably, the immune disorder and/or inflammatory disorder is a GvHD and/or the use in the prevention and/or treatment of the GvHD in the subject comprises a topical application of the composition to an organ or tissue affected by the GvHD, wherein the organ or tissue is at least one of: skin, mucosa, mouth, eyes, lung.

Preferably, the composition comprising platelet derived extracellular vesicles can be a topical pharmaceutical composition, e.g., a cream, a foam, a gel, a lotion, an ointment, for example, a topical pharmaceutical composition as defined herein. Preferably, the use in the prevention and/or treatment of an immune disorder and/or of an inflammatory disorder in a subject comprises a topical application of the composition to an area of skin affected by the immune disorder and/or inflammatory disorder. Preferably, the immune disorder and/or inflammatory disorder is a GvHD and/or the use in the prevention and/or treatment of a GvHD in the subject comprises a topical application of the composition to an area of the skin affected by the GvHD.

Preferably, the composition comprising platelet derived extracellular vesicles can be a topical pharmaceutical composition, e.g., a cream, a foam, a gel, a lotion, an ointment, for example, a topical pharmaceutical composition as defined herein. Preferably, the use in the prevention and/or treatment of an immune disorder and/or of an inflammatory disorder in a subject comprises a topical application of the composition to a mucosa or to an area of a mucosa affected by the immune disorder and/or inflammatory disorder. Preferably, the immune disorder and/or inflammatory disorder is a GvHD and/or the use in the prevention and/or treatment of a GvHD in the subject comprises a topical application of the composition to the mucosa or to the area of the mucosa affected by the GvHD.

Preferably, the composition comprising platelet derived extracellular vesicles can be an ophthalmic composition, for example, an ophthalmic composition as defined herein. Preferably, the use in the prevention and/or treatment of an immune disorder and/or of an inflammatory disorder in a subject comprises a topical application of the composition to an eye affected by the immune disorder and/or inflammatory disorder. Preferably, the immune disorder and/or inflammatory disorder is a GvHD and/or the use in the prevention and/or treatment of a GvHD in the subject comprises a topical application of the composition to an eye affected by the GvHD.

Preferably, the composition comprising platelet derived extracellular vesicles can be an inhalable composition, for example, an inhalable composition as defined herein. Preferably, the use in the prevention and/or treatment of an immune disorder and/or of an inflammatory disorder in a subject comprises a topical application of the composition to the subject's lung affected by the immune disorder and/or inflammatory disorder. Preferably, the immune disorder and/or inflammatory disorder is a GvHD and/or the use in the prevention and/or treatment of a GvHD in the subject comprises a topical application of the composition to the subject's lung affected by the GvHD.

The use in the prevention and/or treatment of the immune disorder and/or inflammatory disorder in the subject may, for example, be a use in the prevention of a GvHD in the subject.

According to the invention, the subject may, for example, be a subject having received hematopoietic stem cells that are not fully consistent with regard to HLA features. Alternatively, the use in the prevention and/or treatment of the immune disorder and/or inflammatory disorder in the subject is a use in the prevention of a GvHD in the subject and the subject will receive hematopoietic stem cells that are not fully consistent with regard to HLA features, preferably within the next 5 days. The subject that will receive the hematopoietic stem cells that are not fully consistent with regard to HLA features may, for example, be a subject scheduled for receiving the hematopoietic stem cells, preferably within the next 5 days.

Preferably, the hematopoietic stem cells that are not fully consistent with regard to HLA features are from a donor who is not parent, not child, not sibling, and not twin of the recipient.

According to the invention, a first drug of the at least two drugs can, for example, be selected from: cyclosporine and tacrolimus, and a second drug of the at least two drugs can, for example, be selected from methotrexate and mycophenolate mofetil. If the first drug is cyclosporine, a content of cyclosporine in the blood of the subject may, for example, be at most 150 ng/ml, e.g., at most 125 ng/ml. If the first drug is tacrolimus, a trough level of tacrolimus in the blood of the subject may, for example, be at most 6 ng/ml, e.g., at most 5 ng/ml.

Preferably, at least one of the at least one or two drugs is selected, e.g., at least two of the at least two drugs are selected, from the following drugs:
- alemtuzumab, interferon beta-1a, interferon beta-1b, peginterferon beta-1a, cladribine, fumarates, e.g., dimethyl fumarate and/or diroximel fumarate, fingolimod, glatiramer acetate, Copolymer 1, Cop-1, mitoxantrone, natalizumab, ocrelizumab, ofatumumab, ozanimod, ponesimod, siponimod, teriflunomide;
and/or
the immune disorder and/or inflammatory disorder is:
- multiple sclerosis.

Preferably, at least one of the at least one or two drugs is selected, e.g., at least two of the at least two drugs are selected, from the following drugs:
- methotrexate;
and/or the immune disorder and/or inflammatory disorder is:
- arthritis, in particular rheumatoid arthritis or juvenile idiopathic arthritis.

Preferably, at least one of the at least one or two drugs is selected, e.g., at least two of the at least two drugs are selected, from the following drugs:
- prednisolone, cyclosporine, tacrolimus, sirolimus, methotrexate, ruxolitinib, infliximab, mycophenolate mofetil;
and/or
the immune disorder and/or inflammatory disorder is:
- a GvHD.

### Specific embodiments:

1. A composition comprising platelet derived extracellular vesicles for use in the prevention and/or treatment of an immune disorder and/or of an inflammatory disorder in a subject, wherein the composition is administered to the subject, wherein
   - the composition is administered to the subject in combination with at least one drug, wherein the at least one drug is selected from the group of immunosuppressive and/or anti-inflammatory drugs
      and/or
   - the immune disorder and/or inflammatory disorder is selected from the group of
      - a graft-versus-host disease,
      - a transplant rejection, e.g., a rejection of a transplanted organ or tissue, in particular a host-versus-graft disease,
      - autoimmune disorders, e.g.,
         - autoimmune intestinal or bowel disorders, e.g., Crohn's disease or ulcerative colitis,
         - autoimmune skin and/or ocular disorders, e.g., psoriasis, vitiligo, atopic dermatitis, uveitis, in particular autoimmune uveitis, for example refractory autoimmune uveitis,
         - autoimmune musculoskeletal disorders, e.g., arthritis, in particular rheumatoid arthritis or juvenile idiopathic arthritis, ankylosing spondylitis, autoimmune myositis, autoimmune polychondritis,
         - autoimmune connective tissue diseases, e.g., Sjögren's syndrome, lupus erythematosus, in particular systemic lupus erythematosus, systemic sclerosis, in particular systemic sclerosis-associated interstitial lung disease, autoimmune vasculitis,
         - neuroinflammation-associated neurodegenerative disorders, e.g., Alzheimer's disease, Parkinson's disease, amyotrophic lateral sclerosis, neurodegenerative disorders, multiple sclerosis,
         - other autoimmune disorders, e.g., autoimmune hepatitis, type 1 diabetes mellitus, lupus nephritis, myasthenia gravis, Grave's disease, and
      - low-grade inflammation and chronic low-grade inflammation, disorders associated with low-grade inflammation and chronic low-grade inflammation, e.g., myalgic encephalomyelitis, chronic fatigue syndrome, myalgic encephalomyelitis/chronic fatigue syndrome, post-viral fatigue syndrome, post-COVID syndrome, adverse reactions after COVID-19 vaccination.
2. The composition for use according to embodiment 1,
   wherein
   - the composition is administered to the subject in combination with at least one drug, wherein the at least one drug is selected from the group of immunosuppressive and/or anti-inflammatory drugs
      and
   - the immune disorder and/or inflammatory disorder is selected from the group of
      - a graft-versus-host disease,
      - a transplant rejection, e.g., a rejection of a transplanted organ or tissue, in particular a host-versus-graft disease,
      - autoimmune disorders, e.g.,
         - autoimmune intestinal or bowel disorders, e.g., Crohn's disease or ulcerative colitis,
         - autoimmune skin and/or ocular disorders, e.g., psoriasis, vitiligo, atopic dermatitis, uveitis, in particular autoimmune uveitis, for example refractory autoimmune uveitis,
         - autoimmune musculoskeletal disorders, e.g., arthritis, in particular rheumatoid arthritis or juvenile idiopathic arthritis, ankylosing spondylitis, autoimmune myositis, autoimmune polychondritis,
         - autoimmune connective tissue diseases, e.g., Sjögren's syndrome, lupus erythematosus, in particular systemic lupus erythematosus, systemic sclerosis, in particular systemic sclerosis-associated interstitial lung disease, autoimmune vasculitis,
         - neuroinflammation-associated neurodegenerative disorders, e.g., Alzheimer's disease, Parkinson's disease, amyotrophic lateral sclerosis, neurodegenerative disorders, multiple sclerosis,
         - other autoimmune disorders, e.g., autoimmune hepatitis, type 1 diabetes mellitus, lupus nephritis, myasthenia gravis, Grave's disease, and
      - low-grade inflammation and chronic low-grade inflammation, disorders associated with low-grade inflammation and chronic low-grade inflammation, e.g., myalgic encephalomyelitis, chronic fatigue syndrome, myalgic encephalomyelitis/chronic fatigue syndrome, post-viral fatigue syndrome, post-COVID syndrome, adverse reactions after COVID-19 vaccination.
3. The composition for use according to embodiment 1 or 2, wherein the at least one drug which is selected from the group of immunosuppressive and/or anti-inflammatory drugs comprises at least one immunosuppressive and/or anti-inflammatory drug which is not prednisolone, e.g., not a corticosteroid.
4. The composition for use according to any one of the preceding embodiments, wherein the composition is administered to the subject in combination with at least two drugs, wherein the at least two drugs are selected from the group of immunosuppressive and/or anti-inflammatory drugs.
5. The composition for use according to embodiment 4, wherein the at least two drugs which are selected from the group of immunosuppressive and/or anti-inflammatory drugs comprise at least one immunosuppressive and/or anti-inflammatory drug which is not prednisolone, e.g., not a corticosteroid.
6. The composition for use according to any one of the preceding embodiments, wherein at least one of the at least one or two drugs is selected, preferably at least two of the at least two drugs are selected, from the following drugs:
   - calcineurin inhibitors, e.g., cyclosporine, aerosolized cyclosporine, voclosporin, tacrolimus, pimecrolimus,
   - inhibitors of nucleotide synthesis and/or antimetabolites and/or nucleoside or nucleobase analogues, e.g., methotrexate, mycophenolate mofetil, mycophenolate sodium, mizoribine, azathioprine, leflunomide, teriflunomide, cladribine,
   - kinase inhibitors, in particular JAK inhibitors, e.g., ruxolitinib, abrocitinib, oclacitinib, filgotinib, baricitinib, peficitinib, upadacitinib, fedratinib, delgocitinib tofacitinib, pacritinib, deucravacitinib, ritlecitinib, deucravacitinib,
   - mTOR inhibitors, e.g., sirolimus, everolimus, temsirolimus, ridaforolimus, umirolimus, zotarolimus,
   - alkylating agents, e.g., cyclophosphamide, chlorambucil,
   - agents suppressing T cell co-stimulation, e.g., abatacept, belatacept,
   - polyclonal and monoclonal antibodies against lymphocyte cell surface antigens, in particular against B lymphocyte and/or T lymphocyte cell surface antigens, e.g., alemtuzumab, natalizumab, ocrelizumab, ofatumumab
   - deoxyspergualin,
   - interferons, e.g., interferon beta-1a, interferon beta-1b, peginterferon beta-1a,
   - fingolimod, myriocin, ozanimod, ponesimod, siponimod,
   - glatiramer acetate, Copolymer 1, Cop-1,
   - mitoxantrone, pixantrone, losoxantrone,
   - stem cell derived extracellular vesicles,
   - corticosteroids, e.g., clobetasol, halobetasol, hydrocortisone, triamcinolone, betamethasone, fluocinole, fluocinonide, prednisone, prednisolone, methylprednisolone,
   - non-steroidal anti-inflammatory drugs, e.g., sulfasalazine, mesalamine, celecoxib, diclofenac, etodolac, fenprofen, flurbiprofen, ibuprofen, ketoprofen, meclofamate, meloxicam, nabumetone, naproxen, oxaprozin, piroxicam, rofecoxib, salicylates, sulindac, tolmetin,
   - phosphodiesterase-4 inhibitors, e.g., rolipram,
   - anti-inflammatory antibodies and/or TNF-alpha inhibitors, e.g., adalimumab, infliximab, etanercept,
   - fumarates, e.g., dimethyl fumarate, diroximel fumarate.
7. The composition for use according to any one of the preceding embodiments, wherein at least one of the at least one or two drugs is selected, preferably at least two of the at least two drugs are selected, from the following drugs:
   - calcineurin inhibitors, e.g., cyclosporine, aerosolized cyclosporine, voclosporin, tacrolimus, pimecrolimus,
   - inhibitors of nucleotide synthesis and/or antimetabolites and/or nucleoside or nucleobase analogues, e.g., methotrexate, mycophenolate mofetil, mycophenolate sodium, mizoribine, azathioprine, leflunomide, teriflunomide, cladribine,
   - kinase inhibitors, in particular JAK inhibitors, e.g., ruxolitinib, abrocitinib, oclacitinib, filgotinib, baricitinib, peficitinib, upadacitinib, fedratinib, delgocitinib tofacitinib, pacritinib, deucravacitinib, ritlecitinib, deucravacitinib,
   - mTOR inhibitors, e.g., sirolimus, everolimus, temsirolimus, ridaforolimus, umirolimus, zotarolimus,
   - anti-inflammatory antibodies and/or TNF-alpha inhibitors, e.g., adalimumab, infliximab, etanercept.
8. The composition for use according to any one of embodiments 4 to 7, wherein at least two of the at least two drugs are selected from:
   - calcineurin inhibitors, e.g., cyclosporine, aerosolized cyclosporine, voclosporin, tacrolimus, pimecrolimus,
   - inhibitors of nucleotide synthesis and/or antimetabolites and/or nucleoside or nucleobase analogues, e.g., methotrexate, mycophenolate mofetil, mycophenolate sodium, mizoribine, azathioprine, leflunomide, teriflunomide, cladribine,
   - kinase inhibitors, in particular JAK inhibitors, e.g., ruxolitinib, abrocitinib, oclacitinib, filgotinib, baricitinib, peficitinib, upadacitinib, fedratinib, delgocitinib tofacitinib, pacritinib, deucravacitinib, ritlecitinib, deucravacitinib,
   - mTOR inhibitors, e.g., sirolimus, everolimus, temsirolimus, ridaforolimus, umirolimus, zotarolimus.
9. The composition for use according to any one of the preceding embodiments, wherein
   - the composition and at least one of the at least one drug or
   - the composition and at least one or at least two of the at least two drugs may be administered to the subject sequentially, in either order, or simultaneously.
10. The composition for use according to any one of the preceding embodiments, wherein the immune disorder and/or inflammatory disorder is accompanied by an excessive immune response.
11. The composition for use according to any one of the preceding embodiments, wherein the immune disorder and/or inflammatory disorder is a graft-versus-host disease, e.g., an acute graft-versus-host disease, a chronic graft-versus-host disease, or a graft-versus-host disease characterized by the simultaneous presence of acute and chronic graft-versus-host disease features.
12. The composition for use according to any one of the preceding embodiments, preferably according to embodiment 11, wherein the immune disorder and/or inflammatory disorder, preferably the graft-versus-host disease, involves or is accompanied by an inflammation-associated ocular and/or corneal disease, e.g., a graft-versus-host disease-associated dry eye disease and/or an uveitis, e.g., an autoimmune uveitis.
13. The composition for use according to embodiment 11 or 12, wherein the graft-versus-host disease is an acute graft-versus-host disease,
   wherein
   - the subject may, in particular, have an acute graft-versus-host disease clinical grading of I, II, III or IV,
   - the subject may, preferably, have an acute graft-versus-host disease clinical grading of II, III or IV,
   - the subject may, most preferably, have an acute graft-versus-host disease clinical grading of III or IV,
   - the subject may, for example, have an acute graft-versus-host disease clinical grading of IV,
      and/or
      wherein the acute graft-versus-host disease may, in particular, be
   - a classic acute graft-versus-host disease,
   - a persistent acute graft-versus-host disease,
   - a recurrent acute graft-versus-host disease, or
   - a late-onset acute graft-versus-host disease.
14. The composition for use according to embodiment 11 or 12, wherein the graft-versus-host disease is a chronic graft-versus-host disease,
   wherein preferably a global severity level of the chronic graft-versus-host disease may be
   - mild, moderate, or severe,
   - most preferably moderate, or severe,
   - e.g., severe,
      and/or
      wherein the chronic graft-versus-host disease may, in particular, be
   - a classic chronic graft-versus-host disease,
   - a progressive chronic graft-versus-host disease,
   - a quiescent chronic graft-versus-host disease, or
   - a de novo chronic graft-versus-host disease.
15. The composition for use according to any one of the preceding embodiments, e.g., according to any one of embodiments 11 to 14, wherein the immune disorder and/or inflammatory disorder, e.g., the graft-versus-host disease, affects one or more of the following organs or tissues: skin, gut, intestine, liver, mucosa, mouth, eyes, lung, heart, and/or gastrointestinal system.
16. The composition for use according to any one of the preceding embodiments,
   wherein the composition is suitable for
   - intravenous administration,
   - infusion,
   - intraperitoneal injection,
   - subcutaneous injection,
   - intra bone injection,
   - intracerebroventricular injection,
   - intra muscular injection,
   - intraocular injection,
   - intra-articular injection or periarticular injection,
   - peritendinous injection,
   - intrathecal injection, or
   - topical administration, e.g.,
      - topical administration to the skin,
      - topical administration to a mucous membrane,
      - ophthalmic administration, e.g. eye drops,
      - inhalational topic administration, or
      - intraoral, periodontal, or interdental application.
17. The composition for use according to any one of the preceding embodiments, wherein the use in the prevention and/or treatment of the immune disorder and/or inflammatory disorder in the subject is a use in the prevention of a graft-versus-host disease in the subject.
18. The composition for use according to any one of the preceding embodiments, in particular according to any one of embodiments 11 to 17,
   wherein
   - the subject has received hematopoietic stem cells that are not fully consistent with regard to HLA features, or
   - the use in the prevention and/or treatment of the immune disorder and/or inflammatory disorder in the subject is a use in the prevention of a graft-versus-host disease in the subject and the subject will receive hematopoietic stem cells that are not fully consistent with regard to HLA features, preferably within the next 5 days.
19. The composition according to any one of the preceding embodiments, wherein the platelet derived extracellular vesicles are *human* platelet lysate derived extracellular vesicles.
20. The composition according to any one of the preceding embodiments 4 to 19, e.g., according to any one of embodiments 11 to 19,
   wherein a first drug of the at least two drugs is selected from:
   - cyclosporine and tacrolimus, and
      wherein a second drug of the at least two drugs is selected from
   - methotrexate and mycophenolate mofetil.
21. The composition according to embodiment 20,
   wherein
   - if the first drug is cyclosporine, a content of cyclosporine in the blood of the subject is at most 150 ng/ml, e.g., at most 125 ng/ml, or
   - if the first drug is tacrolimus, a trough level of tacrolimus in the blood of the subject is at most 6 ng/ml, e.g., at most 5 ng/ml.
22. A composition comprising platelet derived extracellular vesicles and at least one or at least two drugs, wherein at least one drug of the at least one or at least two drugs may preferably be selected from the group of immunosuppressive and/or anti-inflammatory drugs, e.g., from the following drugs:
   - calcineurin inhibitors, e.g., cyclosporine, aerosolized cyclosporine, voclosporin, tacrolimus, pimecrolimus,
   - inhibitors of nucleotide synthesis and/or antimetabolites and/or nucleoside or nucleobase analogues, e.g., methotrexate, mycophenolate mofetil, mycophenolate sodium, mizoribine, azathioprine, leflunomide, teriflunomide, cladribine,
   - kinase inhibitors, in particular JAK inhibitors, e.g., ruxolitinib, abrocitinib, oclacitinib, filgotinib, baricitinib, peficitinib, upadacitinib, fedratinib, delgocitinib tofacitinib, pacritinib, deucravacitinib, ritlecitinib, deucravacitinib,
   - mTOR inhibitors, e.g., sirolimus, everolimus, temsirolimus, ridaforolimus, umirolimus, zotarolimus,
   - alkylating agents, e.g., cyclophosphamide, chlorambucil,
   - agents suppressing T cell co-stimulation, e.g., abatacept, belatacept,
   - polyclonal and monoclonal antibodies against lymphocyte cell surface antigens, in particular against B lymphocyte and/or T lymphocyte cell surface antigens, e.g., alemtuzumab, natalizumab, ocrelizumab, ofatumumab
   - deoxyspergualin,
   - interferons, e.g., interferon beta-1a, interferon beta-1b, peginterferon beta-1a,
   - fingolimod, myriocin, ozanimod, ponesimod, siponimod,
   - glatiramer acetate, Copolymer 1, Cop-1,
   - mitoxantrone, pixantrone, losoxantrone,
   - stem cell derived extracellular vesicles,
   - corticosteroids, e.g., clobetasol, halobetasol, hydrocortisone, triamcinolone, betamethasone, fluocinole, fluocinonide, prednisone, prednisolone, methylprednisolone,
   - non-steroidal anti-inflammatory drugs, e.g., sulfasalazine, mesalamine, celecoxib, diclofenac, etodolac, fenprofen, flurbiprofen, ibuprofen, ketoprofen, meclofamate, meloxicam, nabumetone, naproxen, oxaprozin, piroxicam, rofecoxib, salicylates, sulindac, tolmetin,
   - phosphodiesterase-4 inhibitors, e.g., rolipram,
   - anti-inflammatory antibodies and/or TNF-alpha inhibitors, e.g., adalimumab, infliximab, etanercept,
   - fumarates, e.g., dimethyl fumarate, diroximel fumarate.

Further details are provided in the Figures and in the following non-limiting examples.

### Figures:

- Fig. 1-4:: Bar graphs indicating beneficial effects for new treatments combining platelet derived extracellular vesicles with established immunosuppressive and/or anti-inflammatory drugs
- Fig. 5:: Screening-analysis of relevant T cell-secreted cytokine-profiles
- Fig. 6-10:: Dot plots obtained by flow-cytometric analysis of T cells 4 days after activation under different conditions

### Examples

### 1. Experimental setup

### 1.1 Isolation of platelet derived extracellular vesicles

Excess thrombocyte concentrates from 20 healthy donors were provided by a university hospital, after storage for 5 days at 25 °C, and were used as a source material. Platelet lysates were produced by simple freezing and thawing cycles and centrifugation. Fractions of *human* platelet lysate derived extracellular vesicles (hPLEV-F) were obtained by several steps including filtering, differential centrifugation and PEG-precipitation, i.e., by applying techniques described in detail in EP 3 468 568 B1.

### 1.2 Characterization of platelet derived extracellular vesicles

The extracellular vesicles were characterized in accordance to ISEV-criteria using a nanoparticle tracking analysis, electron microscopy and Western Blots. The extracellular vesicles of the hPLEV-F had a size from 70 to 170 nm and characteristic marker proteins were present.

### 1.3 Functional in vitro assay

A functional *in vitro* assay was performed for proof of beneficial immunotherapeutic effects of combination treatments. Peripheral mononuclear cells (PBMCs) were obtained by density-gradient centrifugation from buffy coat products of different healthy donors. For following experiments, untouched *human* T cells (=CD3+) were isolated by magnetic cell sorting (MACS) using a standard commercial Pan T Cell Isolation Kit and resuspended in culture media (RPMI, 5% *human* AB-serum, 1% Penicillin-Streptomycin). Afterwards T cells were seeded into pretreated 96-well tissue-culture plates (200 000 cells/well/200 µl) and were activated specifically via the T cell receptor by plate-bound anti-CD3 ([5 µg/mL]; clone OKT3) in combination with soluble anti-CD28 ([2 µg/mL]; clone CD28.2). Suitable concentrations of drugs were determined by titration in advance, whereby attention was paid to achieve only partial effects on cellular inhibition. For final experiments, a number of immunosuppressive drugs (prednisolone, cyclosporine, tacrolimus, sirolimus, methotrexate, ruxolitinib, infliximab, mycophenolate mofetil, cyclophosphamide) - each representative for a pharmaceutical subclass - was added in presence or absence of 50 µg *h*PLEV-F per well (=[250 µg/ml]. Unstimulated and stimulated cells as well as stimulated cells with either only *h*PLEV-F or a drug were run as control-samples in parallel. Each condition was set up either in technical duplicates or triplicates.

### 1.4. Analysis of cellular proliferation and activation status

For analysis of cellular proliferation and activation status, cells were analyzed on day 4 after antibody-induced activation by flow-cytometry (Novocyte Advanteon, Agilent). Cell proliferation was documented via size and complexity/granularity (FSC/SSC; forward-scatter/side-scatter) and activation-status via increased CD25-expression. For discrimination of living/dead cells, DAPI (4',6-diamidino-2-phenylindole) was used. Percentage proportions of proliferating/resting and activated/non-activated T cells (LIVE-Gate) were determined and normalized on values of stimulated control samples. Final results were depicted in bar graphs for comparison of either drug-mediated or *h*PLEV-mediated effects alone, or effects mediated by a combination of both, drug and *h*PLEVs.

### 1.5. Analysis of cytokine secretion

For analysis of cytokine secretion after TCR-specific T cell activation, cells were cultured as described before and media supernatants were collected 48 h after activation of T cells including unstimulated samples as a control. A commercial cytokine array was used to capture secreted pro- and anti-inflammatory cytokine profiles from undiluted samples (media supernatant) on a membrane precoated with membrane-bound antibodies. Array images were obtained using a CCD camera-system (Fusion Solo 6S Edge, Vilber Lourmat) with an ideal exposure time of 5 seconds at high sensitivity camera mode. Relevant cytokines were identified and the ELISA-technique was used for quantification of selected cytokines in further experiments.

### 2. Results

### 2.1 Inhibition of T cell proliferation and of T cell activation

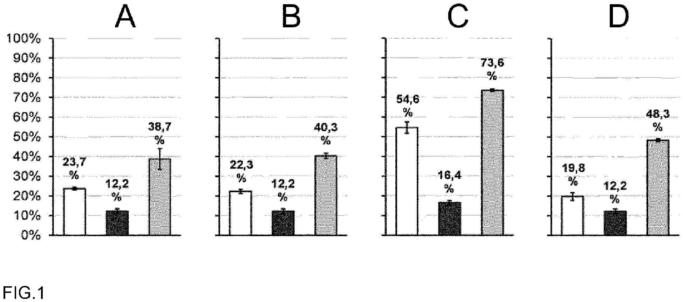

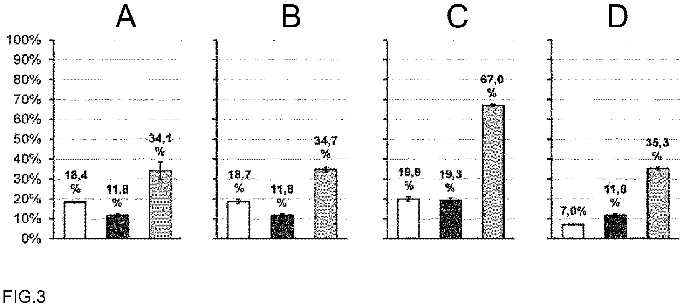

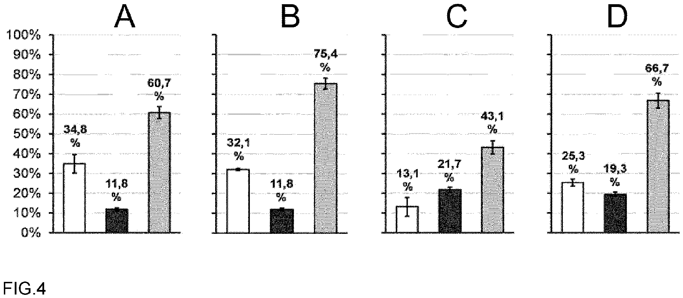

Figures 1 to 4 show beneficial effects on activated T cells for combination treatments using well-known immunosuppressive and/or anti-inflammatory drugs together with hPLEVs. Experiments were performed as described hereinabove under "1. Experimental setup". Activated cells were analyzed on day 4 by flow-cytometry. Living T cells were analyzed for two experimentally relevant parameters, the TCR-receptor induced proliferation via FSC/SSC (Fig. 1 and 2) and the activation status via CD25 expression (Fig. 3 and 4).

The bar graphs in Fig. 1 and 2 show amounts of resting T cells. The bar graphs in Fig. 3 and 4 show amounts of non-activated T cells, based on CD25-expression. In Fig. 1 to 4, white bars show results obtained in the presence of immunosuppressive and/or anti-inflammatory drug alone, black bars show results obtained in the presence of platelet derived extracellular vesicles alone, and grey bars show results obtained in the presence of a combination of both. The experiments represented in Fig. 1 to 4 were carried out with eight different drugs, as indicated below in table 1. Cyclophosphamide alone turned out to be cytotoxic, so that no results could be obtained.

**Table 1: Immunosuppressive and/or anti-inflammatory drugs and platelet derived extracellular vesicles tested alone and in combination**

| Figure Panel | white bar | black bar | grey bar |
|---|---|---|---|
| 1A and 3A | prednisolone | EVs | prednisolone and EVs |
| 1B and 3B | cyclosporine | EVs | cyclosporine and EVs |
| 1C and 3C | tacrolimus | EVs | tacrolimus and EVs |
| 1D and 3D | sirolimus | EVs | sirolimus and EVs |
| 2A and 4A | methotrexate | EVs | methotrexate and EVs |
| 2B and 4B | ruxolitinib | EVs | ruxolitinib and EVs |
| 2C and 4C | infliximab | EVs | infliximab and EVs |
| 2D and 4D | mycophenolate mofetil | EVs | mycophenolate mofetil and EVs |

These drugs are representative of different established subclasses of immunosuppressive and/or anti-inflammatory drugs.

All data are normalized on the corresponding, stimulated control samples.

Fig. 1 to 4 show that all drugs alone as well as the platelet derived extracellular vesicles alone inhibit both, the proliferation and activation of induced T cells. The strongest immunosuppressive outcomes are achieved in presence of the combinations of both. The combinations provide an additive or even an unexpected super-additive (synergistic) reinforcement of the effects in both, or at least in one of the analyzed parameters (inhibition of proliferation as shown in Fig. 1 and 2, inhibition of activation as shown in Fig. 3 and 4). Results are summarized below in tables 2 to 9:

**Table 2: Inhibition of T cell proliferation and of T cell activation by prednisolone, by platelet derived extracellular vesicles, and by a combination of both**

| **Analyzed parameter 1: Cell proliferation** | | | |
|---|---|---|---|
| **Resting T cells** | **X̅** | **σ** | **Combined effects** |
| **prednisolone** | 23,7% | 0,7% | Additive effects on inhibition of prolif. |
| **EVs** | 12,2% | 1,3% | |
| **combination** | 38,7% | 5,3% | |

| **Analyzed parameter 2:** | | | |
|---|---|---|---|
| **Cell activation via CD25 expression** | | | |
| **Non-activ. T cells** | **X̅** | **σ** | **Combined effects** |
| **prednisolone** | 18,4% | 0,4% | Additive effects on inhibition of activ. |
| **EVs** | 11,8% | 0,7% | |
| **combination** | 34,1% | 4,5% | |

**Table 3: Inhibition of T cell proliferation and of T cell activation by cyclosporine, by platelet derived extracellular vesicles, and by a combination of both**

| **Analyzed parameter 1: Cell proliferation** | | | |
|---|---|---|---|
| **Resting T cells** | **X̅** | **σ** | **Combined effects** |
| **cyclosporine** | 22,3% | 1,0% | Slightly overadditive effects on inhibition of prolif. (ca. 5%) |
| **EVs** | 12,2% | 1,3% | |
| **combination** | 40,3% | 1,3% | |

| **Analyzed parameter 2: Cell activation via CD25 expression** | | | |
|---|---|---|---|
| **Non-activ. T cells** | **X̅** | **σ** | **Combined effects** |
| **cyclosporine** | 18,7% | 1,0% | Slightly overadditive effects on inhibition of activ. (ca. 5%) |
| **EVs** | 11,8% | 0,7% | |
| **combination** | 34,7% | 1,3% | |

**Table 4: Inhibition of T cell proliferation and of T cell activation by tacrolimus, by platelet derived extracellular vesicles, and by a combination of both**

| **Analyzed parameter 1: Cell proliferation** | | | |
|---|---|---|---|
| **Resting T cells** | **X̅** | **σ** | **Combined effects** |
| **tacrolimus** | 54,6% | 2,9% | Additive effects on inhibition of prolif. |
| **EVs** | 16,4% | 1,2% | |
| **combination** | 73,6% | 0,6% | |

| **Analyzed parameter 2: Cell activation via CD25 expression** | | | |
|---|---|---|---|
| **Non-activ. T cells** | **X̅** | **σ** | **Combined effects** |
| **tacrolimus** | 19,9% | 1,1% | Strong overadditive effects on inhibition of activ. (ca. 28%) |
| **EVs** | 19,3% | 1,1% | |
| **combination** | 67,0% | 0,5% | |

**Table 5: Inhibition of T cell proliferation and of T cell activation by sirolimus, by platelet derived extracellular vesicles, and by a combination of both**

| **Analyzed parameter 1: Cell proliferation** | | | |
|---|---|---|---|
| **Resting T cells** | **X̅** | **σ** | **Combined effects** |
| **sirolimus** | 19,8% | 1,9% | Clear overadditive effects on inhibition of prolif. (ca. 16,5%) |
| **EVs** | 12,2% | 1,3% | |
| **combination** | 48,3% | 0,7% | |

| **Analyzed parameter 2: Cell activation via CD25 expression** | | | |
|---|---|---|---|
| **Non-activ. T cells** | **X̅** | **σ** | **Combined effects** |
| **sirolimus** | 7,0% | 0,1% | Clear overadditive effects on inhibition of activ. (ca. 16,5%) |
| **EVs** | 11,8% | 0,7% | |
| **combination** | 35,3% | 0,8% | |

**Table 6: Inhibition of T cell proliferation and of T cell activation by methotrexate, by platelet derived extracellular vesicles, and by a combination of both**

| **Analyzed parameter 1: Cell proliferation** | | | |
|---|---|---|---|
| **Resting T cells** | **X̅** | **σ** | **Combined effects** |
| **methotrexate** | 47,7% | 2,6% | Clear overadditive effects on inhibition of prolif. (ca. 10%) |
| **EVs** | 12,2% | 1,3% | |
| **combination** | 69,2% | 3,0% | |

| **Analyzed parameter 2: Cell activation via CD25 expression** | | | |
|---|---|---|---|
| **Non-activ. T cells** | **X̅** | **σ** | **Combined effects** |
| **methotrexate** | 34,8% | 4,6% | Clear overadditive effects on inhibition of activ. (ca. 14%) |
| **EVs** | 11,8% | 0,7% | |
| **combination** | 60,7% | 3,0% | |

**Table 7: Inhibition of T cell proliferation and of T cell activation by ruxolitinib, by platelet derived extracellular vesicles, and by a combination of both**

| **Analyzed parameter 1: Cell proliferation** | | | |
|---|---|---|---|
| **Resting T cells** | **X̅** | **σ** | **Combined effects** |
| **ruxolitinib** | 22,2% | 2,1% | Strong overadditive effects on inhibition of prolif. (ca. 22,5%) |
| **EVs** | 12,2% | 1,3% | |
| **combination** | 56,8% | 3,9% | |

| **Analyzed parameter 2: Cell activation via CD25 expression** | | | |
|---|---|---|---|
| **Non-activ. T cells** | **X̅** | **σ** | **Combined effects** |
| **ruxolitinib** | 32,1% | 0,6% | Strong overadditive effects on inhibition of activ. (ca. 31,5%) |
| **EVs** | 11,8% | 0,7% | |
| **combination** | 75,4% | 2,8% | |

**Table 8: Inhibition of T cell proliferation and of T cell activation by infliximab, by platelet derived extracellular vesicles, and by a combination of both**

| **Analyzed parameter 1: Cell proliferation** | | | |
|---|---|---|---|
| **Resting T cells** | **X̅** | **σ** | **Combined effects** |
| **infliximab** | 14,3% | 3,9% | Slightly overadditive effects on inhibition of prolif. (ca. 6,5%) |
| **EVs** | 22,5% | 1,2% | |
| **combination** | 43,2% | 4,4% | |

| **Analyzed parameter 2: Cell activation via CD25 expression** | | | |
|---|---|---|---|
| **Non-activ. T cells** | **X̅** | **σ** | **Combined effects** |
| **infliximab** | 13,1% | 4,7% | Slightly overadditive effects on inhibition of activ. (ca. 8,5%) |
| **EVs** | 21,7% | 1,3% | |
| **combination** | 43,1% | 3,3% | |

**Table 9: Inhibition of T cell proliferation and of T cell activation by mycophenolate mofetil, by platelet derived extracellular vesicles, and by a combination of both**

| **Analyzed parameter 1: Cell proliferation** | | | |
|---|---|---|---|
| **Resting T cells** | **X̅** | **σ** | **Combined effects** |
| **mycophenolate mofetil** | 43,1% | 5,4% | Clear overadditive effects on inhibition of prolif. (ca. 14,5%) |
| **EVs** | 16,4% | 1,2% | |
| **combination** | 73,8% | 3,0% | |

| **Analyzed parameter 2: Cell activation via CD25 expression** | | | |
|---|---|---|---|
| **Non-activ. T cells** | **X̅** | **σ** | **Combined effects** |
| **mycophenolate mofetil** | 25,3% | 1,8% | Strong overadditive effects on inhibition of activ. (ca. 22%) |
| **EVs** | 19,3% | 1,1% | |
| **combination** | 66,7% | 3,8% | |

The results illustrated in Fig. 1 to 4 and tables 2 to 9 clearly suggest that super-additive effects can be strongest if platelet derived extracellular vesicles are combined with a range of different subclasses of immunosuppressive and/or anti-inflammatory drugs.

These subclasses include in particular:
- kinase inhibitors or JAK inhibitors for which super-additive effects were strongest (as demonstrated with ruxolitinib),
- inhibitors of nucleotide synthesis and/or antimetabolites and/or nucleoside or nucleobase analogues, for which very strong super-additive effects were observed (as demonstrated with methotrexate, mycophenolate mofetil), and
- mTOR inhibitors for which very strong super-additive effects were observed (as demonstrated with sirolimus).

### 2.2 Cytokine profiles

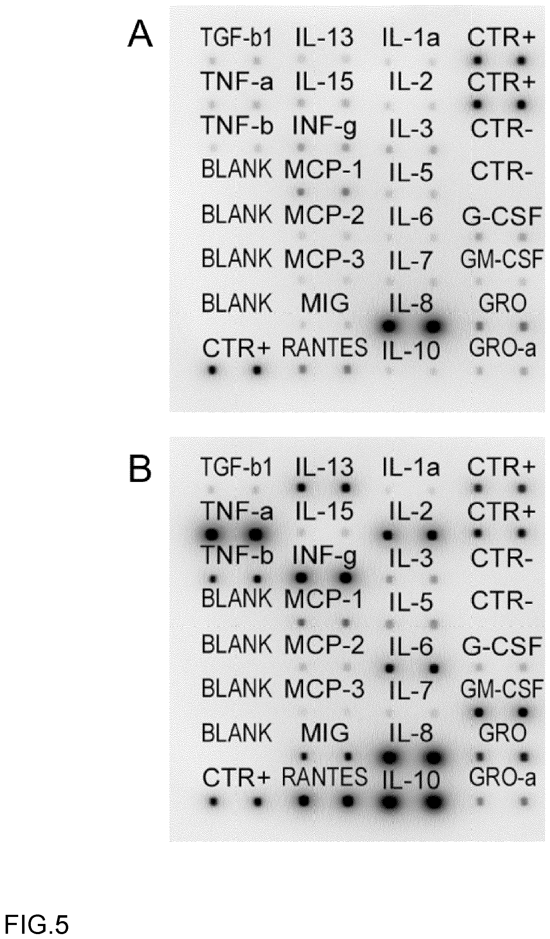

Fig. 5 exemplarily shows cytokine profiles secreted by specifically activated T cells in the established *in vitro* assay. Experiments were performed as described above in sections 1.3 and 1.5. Cell culture supernatants were collected 48 hours after antibody-induced activation of T cells and analyzed for a set of 23 secreted cytokines. Fig. 5B shows the screening-analysis on a cytokine array membrane. Fig. 5A shows the profile of an unstimulated control sample. Captured results of cytokine profiles allow visual comparison as well as densitometric analysis of cytokine-expression levels under chosen experimental conditions.

The following 11 cytokines were found to be increased upon T cell specific activation: GM-CSF, GRO, IL-2, IL-6, IL-10, IL-13, INF-g, MIG, RANTES, TNF-α, TNF-β. These are described in table 10:

**Table 10: Cytokines secreted at increased levels after αCD3/αCD28 induced T cell activation in vitro**

| Cytokine | Description of T cell secreted cytokines |
|---|---|
| GM-CSF | Category: colony stimulating factor |
| | Context: pro-inflammatory |
| | Immune-related function: Stimulating activation + migration of myeloid cells to sites of inflammation (also in chronic inflammatory diseases); promoting survival of target cells and stimulating the renewal of effector granulocytes and macrophages (effect on hematopoiesis) |
| | Pathological relevance: yes, e.g., in autoimmune diseases such as rheumatoid arthritis or multiple sclerosis |
| GRO | Category: CXC-chemokine |
| (CXCL1) | Context: pro-inflammatory |
| | Immune-related function: Chemotactic cytokine, mainly for neutrophil-recruitment at sites of inflammation, monocytes and basophils to a lesser extend; produced by macrophages, neutrophils and Th17 cells (and epithelial cells) upon inflammation; expression induced indirectly by IL-1, TNF-α or IL-17 and triggered mainly by activation of NF-κB or C/EBPβ signaling pathways predominantly involved in inflammation and leading to production of other inflammatory cytokines. |
| | Pathological relevance: yes, e.g., in CNS-pathologies like multiple sclerosis/ brain injury; contribution to prostaglandin-release resulting in increased pain-sensitivity; phosphorylation of ERK1/ERK2 kinases + activation of NMDA receptors leads to transcription of genes (such as c-Fos, COX-2) inducing chronic pain; role in cancer (reinforcement of angiogenesis); |
| IL-2 | Category: Interleukin |
| | Context: Pro- and anti-inflammatory (pleiotropic functions) Immune-related function: Reinforcement of T cell activation + proliferation, activation of NK cells towards autoimmunity but also induction and maintenance of regulatory T cells for keeping immune-homeostasis |
| | Pathological relevance: yes, but not clinically relevant in laboratory diagnostics due to the low concentrations in serum and low plasma half-lives. |
| IL-6 | Category: Interleukin |
| | Context: Pro- and anti-inflammatory (pleiotropic functions) Immune-related function: effect on inflammation, immune response, and hematopoiesis; inducer of the acute-phase response as well as specific cellular and humoral immune responses; inflammatory key cytokine |
| | Pathological relevance: yes, various immune diseases (e.g., rheumatoid arthritis) involve a dysregulated, continual synthesis of IL-6 |
| IL-10 | Category: Interleukin |
| | Context: Anti-inflammatory |
| | Immune-related function: immunoregulation + inhibition of pro-inflammatory cytokines; anti-inflammatory effects on eosinophils, basophils + mast; influence on macrophages + on resting B-cells |
| | Pathological relevance: yes, plays a major role in keeping immune-homeostasis or, e.g., the control and regulation of allergy and asthma. |
| IL-13 | Category: Interleukin |
| | Context: Anti-inflammatory |
| | Immune-related function: immunoregulation + inhibition of pro-inflammatory cytokines; influence on monocytes + macrophages; homologous to IL-4 |
| | Pathological relevance: yes, e.g., plays a significant role in the downregulation of inflammatory processes underlying RA pathology, and beneficially modulate the course of the disease |
| INF-γ | Category: Interferon |
| | Context: Pro-inflammatory |
| | Immune-related function: important role in both innate + adaptive immunity; promotion of Th1 immune responses + secretion of Th1-associated cytokines; influence on macrophages |
| | Pathological relevance: yes, e.g., aberrant/elevated IFN-γ expression is associated with a number of autoimmune diseases |
| MIG (CXCL9) | Category: CXC-chemokine |
| | Context: pro-inflammatory |
| | Immune related function: Chemotactic cytokine for leukocyte recruitment (preferentially of the Th1 phenotype, which express high levels of CXCR3; upregulation of T cell response; regulation of immune cell migration, differentiation, and activation at sites of inflammation or injury; primarily induced |
| | Pathological relevance: yes, e.g., key role of CXCL9 in age-related chronic inflammation; involved in cardiac aging, poor vascular function, and adverse cardiac remodeling; higher levels of CXCL9 are tied to an elevated risk for strokes, kidney failure, and heart attacks |
| | The expression of CXCL9 is induced primarily by the Th1-associated cytokine IFN-γ and correlates with tissue infiltration of T lymphocytes in a number of Th1-associated diseases, suggesting that CXCL9 plays an important role in the regulation of effector cell recruitment to sites of inflammation |
| RANTES (CCL5) | Category: CC-chemokine |
| | Context: pro-inflammatory |
| | Immune related function: Chemotactic cytokine induced by TNF-alpha and IL-1 alpha; activating T cells, basophils, eosinophils, monocytes and other cell in context of leukocyte-recruitment into sites of inflammation |
| | Pathological relevance: yes, elevated levels of RANTES in blood/serum are associated with several systemic and local inflammatory diseases e.g., autoimmune diseases, stroke patients, allergies, asthma, multiple sclerosis, bacterial infection, some tumor diseases. Not preferentially used in medical laboratory diagnostics due to existence of more sensitive markers for systemic inflammation (such as e.g.TNF-α, IP-10, IL-6) |
| TNF-α | Category: tumor necrosis factors |
| | Context: Pro-inflammatory |
| | Immune related function: Inflammatory key cytokine, upregulated during local and systemic inflammation; potent paracrine and endocrine mediator of inflammatory and immune functions; also known to regulate growth and differentiation of a wide variety of cells types |
| | Pathological relevance: yes, functioning as a multifunctional mediator in various local + systemic physiological and pathophysiological conditions affecting: immune system, central nervous system, musculoskeletal system, endothel and adipose tissue, liver and inflammation; elevated levels of TNF-α are associated with increased inflammation; TNF-α is target in many therapies. |
| TNF-β (Lympho-toxin-α) | Category: tumor necrosis factors |
| | Context: Pro-inflammatory |
| | Immune-related function: Inflammatory cytokine, produced/secreted by activated T-lymphocytes; effect is mediated via the same receptors as that of TNF-α; plays important role in immunoregulation |
| | Pathological relevance: yes, e.g., functioning as a multifunctional mediator in immunoregulatory processes during inflammation |

These cytokines play an important role in immune homeostasis under physiological as well as pathophysiological conditions.

TNF-α, IL-6 and IFN-γ belong to the most important proinflammatory marker-cytokines in context of laboratory diagnostics. In general, cytokine-levels of proinflammatory TNF-α, IL-1β, IL-6, IFN-γ and IP-10 are found to be elevated in blood/plasma/serum of patients dealing with local or systemic inflammation. Together with the anti-inflammatory marker cytokines IL-10 and TGF-β, these cytokines are used to analyze patients' immune status and to monitor therapeutic course/progress in patients suffering from e.g., acute or chronic systemic inflammation, silent inflammation, autoimmune diseases (such as rheumatoid arthritis, collagenosis, ulcerative colitis, Crohn's disease, myalgic encephalomyelitis / chronic fatigue syndrome (ME/CFS) and infectious diseases (fungi, bacteria, viruses). This set of cytokines is also considered for analyzing disease-induced tissue destruction and necrosis.

### 2.3 Cell protective effects

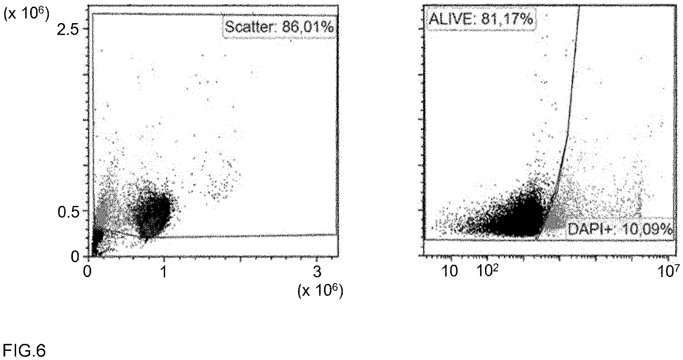

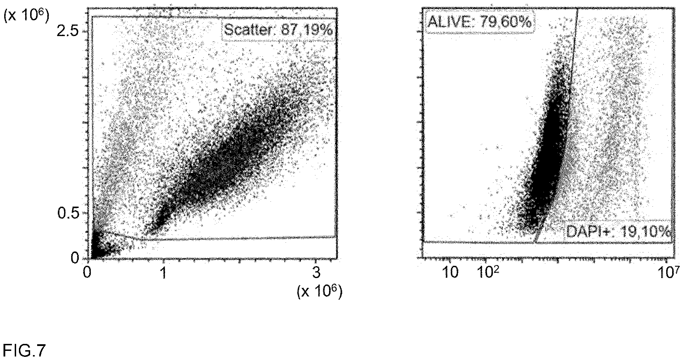

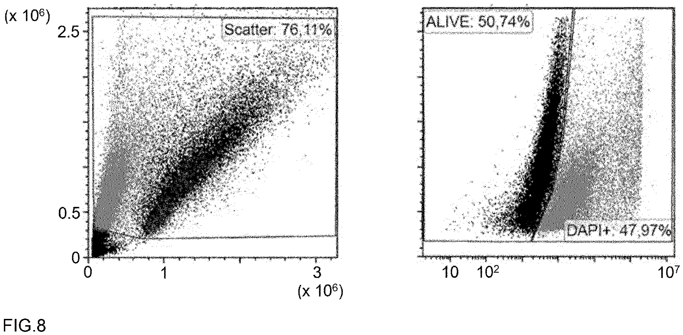

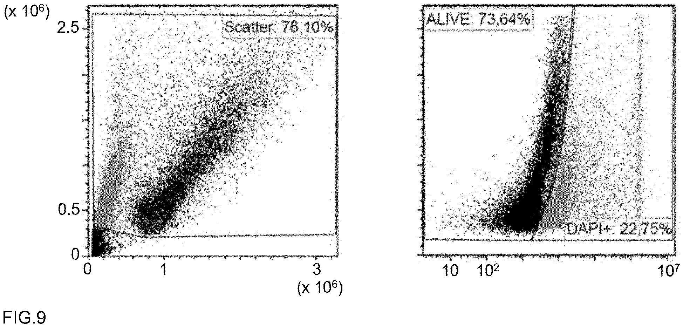

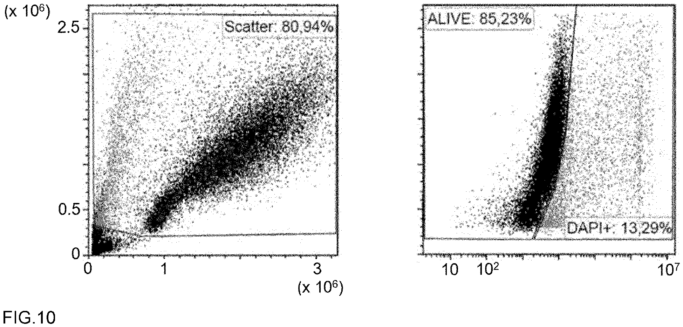

Surprising and relevant cell protective effects on immune-cells were observed in addition to the above-described additive and super-additive effects. Cell protective effects were observed when platelet derived extracellular vesicles were present in addition to methotrexate. T cell-experiments were performed as described above (see "1.3 Functional *in vitro* assay"). Cells were analyzed by flow-cytometry 4 days after T cell activation. Fig. 6 to 10 illustrate cell protective effects on immune-cells using combination treatment of conventional therapeutics together with platelet derived extracellular vesicles. (Fig. 6: T cells that were not activated (unstimulated control), Fig. 7: T cells induced with αCD3/αCD28 (stimulated control), Fig. 8: activated T cells suppressed with methotrexate (stimulated cells + methotrexate), Fig. 9: activated T cells suppressed with platelet derived extracellular vesicles and methotrexate (stimulated cells + methotrexate + EVs), Fig. 10: activated T cells suppressed with platelet derived extracellular vesicles (stimulated cells + EVs)). In all figures 6 to 10, the left dot plot shows size and complexity/granularity (FSC/SSC; forward-scatter/side-scatter) of T cells. The plot includes a scatter-gate including T cells and excluding debris. In all figures 6 to 10, the right dot plot shows the side-scatter signal against fluorescence of DAPI (4',6-diamidino-2-phenylindole), which is used for discrimination of living/dead cells. DAPI-negative cells are living cells (ALIVE-Gate, coloured in black). DAPI-positive cells are coloured in grey.

For the treatment with methotrexate alone, a suitable concentration of methotrexate in a range from 250-375 ng/ml media was used to partially suppress proliferation as well as CD25-expression of specifically activated T cells. In addition to the observed, desired immunosuppressive effects, the drug was found to exert cytotoxic effects. As methotrexate is known to be an effective but cytotoxic drug, this was to be expected.

While inhibitory effects were increased in presence of the combination treatment, cytotoxic effects were surprisingly reduced. The combination treatment turned out to be advantageous over methotrexate alone in terms of cell vitality, because methotrexate-induced cytotoxicity can be compensated. These "rescue-effects" could be demonstrated repeatedly using T cells of different donors in independent experiments.

It was found that 80% of stimulated cells were alive (Fig. 7). In presence of methotrexate, percentages of living cells decreased to only 50% due to cytotoxic effects of the drug (Fig. 8). In presence of both methotrexate and platelet derived extracellular vesicles, cytotoxic effects were obviously compensated by adding the vesicles (Fig. 9). Here percentages of living cells reached 75%. A combination treatment according to the invention thus surprisingly reduced methotrexate-mediated cytotoxicity.

For a more detailed analysis of cytotoxic effects, a subclassification of necrotic phenotypes was performed by a 4-color staining technique including annexin A5-FITC, propidium iodide, DiIC1(5), and Hoechst 33342 according to known literature. This analysis confirmed that samples treated with a combination of methotrexate and platelet derived extracellular vesicles contained less apoptotic and less necrotic cells. Surprisingly, a destruction of cellular mitochondria was reduced by the combination treatment compared to treatment with methotrexate alone.

### 3. Conclusion and discussion

In previous internal preclinical studies, we were able to verify high biocompatibility of platelet derived extracellular vesicles with various cell types in vitro as well as in vivo in animal models. Our platelet derived EV-products are compatible with blood-cells from different donors of all blood-groups. No cytotoxicity on cells of the immune-system was detected so far.

Now, the recent results presented herein show that platelet derived extracellular vesicles are able to exert immunosuppressive effects comparable to those of a broad range of well-known immunosuppressive and/or anti-inflammatory drugs. Furthermore, the combination of platelet derived extracellular vesicles with well-known immunosuppressive and/or anti-inflammatory drugs shows beneficial immunosuppressive effects. Super-additive effects were observed on either proliferation or activation, or on both measured parameters for various combinations with various well-known immunosuppressive and/or anti-inflammatory drugs.

Clinically and diagnostically relevant characteristic proinflammatory cytokines are affected. The examples hereinabove imply that smaller amounts of these cytokines were secreted by immune cells when these were treated with different compositions according to the invention.

Furthermore, cell-protective effects were observed when a composition according to the invention was tested. It was surprising to find increased immunosuppression even though T cell vitality was increased when T cells were treated with platelet derived extracellular vesicles and drug instead of drug alone.

Taken together, the data presented indicate that the invention can help in particular patients suffering from various severe immune disorders and/or from other disorders that are associated with or accompanied by inflammatory conditions. The invention might allow the administration of lower doses of a known immunosuppressive and/or anti-inflammatory drug or of multiple immunosuppressive and/or anti-inflammatory drugs. This can reduce unwanted and harmful side-effects originating from the drug or drugs, while achieving equal or better immunotherapeutic effects. Due to observation of cell-protective effects, a combination therapy might additionally reduce cytotoxic effects of conventional agents providing additional benefit for treated patients.

## Claims

1. A composition comprising platelet derived extracellular vesicles, e.g., *human* platelet lysate derived extracellular vesicles, for use in the prevention and/or treatment of an immune disorder and/or of an inflammatory disorder in a subject, wherein the composition is administered to the subject,
wherein
- the composition is administered to the subject in combination with at least one drug, wherein the at least one drug is selected from the group of immunosuppressive and/or anti-inflammatory drugs
and/or
- the immune disorder and/or inflammatory disorder is selected from the group of
- a graft-versus-host disease,
- a transplant rejection, e.g., a rejection of a transplanted organ or tissue, in particular a host-versus-graft disease,
- autoimmune disorders, e.g.,
- autoimmune intestinal or bowel disorders, e.g., Crohn's disease or ulcerative colitis,
- autoimmune skin and/or ocular disorders, e.g., psoriasis, vitiligo, atopic dermatitis, uveitis, in particular autoimmune uveitis, for example refractory autoimmune uveitis,
- autoimmune musculoskeletal disorders, e.g., arthritis, in particular rheumatoid arthritis or juvenile idiopathic arthritis, ankylosing spondylitis, autoimmune myositis, autoimmune polychondritis,
- autoimmune connective tissue diseases, e.g., Sjögren's syndrome, lupus erythematosus, in particular systemic lupus erythematosus, systemic sclerosis, in particular systemic sclerosis-associated interstitial lung disease, autoimmune vasculitis,
- neuroinflammation-associated neurodegenerative disorders, e.g., Alzheimer's disease, Parkinson's disease, amyotrophic lateral sclerosis, neurodegenerative disorders, multiple sclerosis,
- other autoimmune disorders, e.g., autoimmune hepatitis, type 1 diabetes mellitus, lupus nephritis, myasthenia gravis, Grave's disease, and
- low-grade inflammation and chronic low-grade inflammation, disorders associated with low-grade inflammation and chronic low-grade inflammation, e.g., myalgic encephalomyelitis, chronic fatigue syndrome, myalgic encephalomyelitis/chronic fatigue syndrome, post-viral fatigue syndrome, post-COVID syndrome, adverse reactions after COVID-19 vaccination.

2. The composition for use according to claim 1,
wherein
- the composition is administered to the subject in combination with at least one drug, wherein the at least one drug is selected from the group of immunosuppressive and/or anti-inflammatory drugs
and
- the immune disorder and/or inflammatory disorder is selected from the group of
- a graft-versus-host disease,
- a transplant rejection, e.g., a rejection of a transplanted organ or tissue, in particular a host-versus-graft disease,
- autoimmune disorders, e.g.,
- autoimmune intestinal or bowel disorders, e.g., Crohn's disease or ulcerative colitis,
- autoimmune skin and/or ocular disorders, e.g., psoriasis, vitiligo, atopic dermatitis, uveitis, in particular autoimmune uveitis, for example refractory autoimmune uveitis,
- autoimmune musculoskeletal disorders, e.g., arthritis, in particular rheumatoid arthritis or juvenile idiopathic arthritis, ankylosing spondylitis, autoimmune myositis, autoimmune polychondritis,
- autoimmune connective tissue diseases, e.g., Sjögren's syndrome, lupus erythematosus, in particular systemic lupus erythematosus, systemic sclerosis, in particular systemic sclerosis-associated interstitial lung disease, autoimmune vasculitis,
- neuroinflammation-associated neurodegenerative disorders, e.g., Alzheimer's disease, Parkinson's disease, amyotrophic lateral sclerosis, neurodegenerative disorders, multiple sclerosis,
- other autoimmune disorders, e.g., autoimmune hepatitis, type 1 diabetes mellitus, lupus nephritis, myasthenia gravis, Grave's disease, and
- low-grade inflammation and chronic low-grade inflammation, disorders associated with low-grade inflammation and chronic low-grade inflammation, e.g., myalgic encephalomyelitis, chronic fatigue syndrome, myalgic encephalomyelitis/chronic fatigue syndrome, post-viral fatigue syndrome, post-COVID syndrome, adverse reactions after COVID-19 vaccination.

3. The composition for use according to claim 1 or 2, wherein the at least one drug which is selected from the group of immunosuppressive and/or anti-inflammatory drugs comprises at least one immunosuppressive and/or anti-inflammatory drug which is not prednisolone, e.g., not a corticosteroid.

4. The composition for use according to any one of the preceding claims, wherein the composition is administered to the subject in combination with at least two drugs, wherein the at least two drugs are selected from the group of immunosuppressive and/or anti-inflammatory drugs.

5. The composition for use according to claim 4, wherein the at least two drugs which are selected from the group of immunosuppressive and/or anti-inflammatory drugs comprise at least one immunosuppressive and/or anti-inflammatory drug which is not prednisolone, e.g., not a corticosteroid.

6. The composition for use according to any one of the preceding claims, wherein at least one of the at least one or two drugs is selected, preferably at least two of the at least two drugs are selected, from the following drugs:
- calcineurin inhibitors, e.g., cyclosporine, aerosolized cyclosporine, voclosporin, tacrolimus, pimecrolimus,
- inhibitors of nucleotide synthesis and/or antimetabolites and/or nucleoside or nucleobase analogues, e.g., methotrexate, mycophenolate mofetil, mycophenolate sodium, mizoribine, azathioprine, leflunomide, teriflunomide, cladribine,
- kinase inhibitors, in particular JAK inhibitors, e.g., ruxolitinib, abrocitinib, oclacitinib, filgotinib, baricitinib, peficitinib, upadacitinib, fedratinib, delgocitinib tofacitinib, pacritinib, deucravacitinib, ritlecitinib, deucravacitinib,
- mTOR inhibitors, e.g., sirolimus, everolimus, temsirolimus, ridaforolimus, umirolimus, zotarolimus,
- alkylating agents, e.g., cyclophosphamide, chlorambucil,
- agents suppressing T cell co-stimulation, e.g., abatacept, belatacept,
- polyclonal and monoclonal antibodies against lymphocyte cell surface antigens, in particular against B lymphocyte and/or T lymphocyte cell surface antigens, e.g., alemtuzumab, natalizumab, ocrelizumab, ofatumumab
- deoxyspergualin,
- interferons, e.g., interferon beta-1a, interferon beta-1b, peginterferon beta-1a,
- fingolimod, myriocin, ozanimod, ponesimod, siponimod,
- glatiramer acetate, Copolymer 1, Cop-1,
- mitoxantrone, pixantrone, losoxantrone,
- stem cell derived extracellular vesicles,
- corticosteroids, e.g., clobetasol, halobetasol, hydrocortisone, triamcinolone, betamethasone, fluocinole, fluocinonide, prednisone, prednisolone, methylprednisolone,
- non-steroidal anti-inflammatory drugs, e.g., sulfasalazine, mesalamine, celecoxib, diclofenac, etodolac, fenprofen, flurbiprofen, ibuprofen, ketoprofen, meclofamate, meloxicam, nabumetone, naproxen, oxaprozin, piroxicam, rofecoxib, salicylates, sulindac, tolmetin,
- phosphodiesterase-4 inhibitors, e.g., rolipram,
- anti-inflammatory antibodies and/or TNF-alpha inhibitors, e.g., adalimumab, infliximab, etanercept,
- fumarates, e.g., dimethyl fumarate, diroximel fumarate.

7. The composition for use according to any one of the preceding claims, wherein at least one of the at least one or two drugs is selected, preferably at least two of the at least two drugs are selected, from the following drugs:
- calcineurin inhibitors, e.g., cyclosporine, aerosolized cyclosporine, voclosporin, tacrolimus, pimecrolimus,
- inhibitors of nucleotide synthesis and/or antimetabolites and/or nucleoside or nucleobase analogues, e.g., methotrexate, mycophenolate mofetil, mycophenolate sodium, mizoribine, azathioprine, leflunomide, teriflunomide, cladribine,
- kinase inhibitors, in particular JAK inhibitors, e.g., ruxolitinib, abrocitinib, oclacitinib, filgotinib, baricitinib, peficitinib, upadacitinib, fedratinib, delgocitinib tofacitinib, pacritinib, deucravacitinib, ritlecitinib, deucravacitinib,
- mTOR inhibitors, e.g., sirolimus, everolimus, temsirolimus, ridaforolimus, umirolimus, zotarolimus,
- anti-inflammatory antibodies and/or TNF-alpha inhibitors, e.g., adalimumab, infliximab, etanercept.

8. The composition for use according to any one of the preceding claims, wherein
- the composition and at least one of the at least one drug or
- the composition and at least one or at least two of the at least two drugs may be administered to the subject sequentially, in either order, or simultaneously.

9. The composition for use according to any one of the preceding claims, wherein the immune disorder and/or inflammatory disorder is accompanied by an excessive immune response.

10. The composition for use according to any one of the preceding claims, wherein the immune disorder and/or inflammatory disorder is a graft-versus-host disease, e.g., an acute graft-versus-host disease, a chronic graft-versus-host disease, or a graft-versus-host disease **characterized by** the simultaneous presence of acute and chronic graft-versus-host disease features.

11. The composition for use according to any one of the preceding claims, preferably according to claim 10, wherein the immune disorder and/or inflammatory disorder, preferably the graft-versus-host disease, involves or is accompanied by an inflammation-associated ocular and/or corneal disease, e.g., a graft-versus-host disease-associated dry eye disease and/or an uveitis, e.g., an autoimmune uveitis and/or a keratoconjunctivitis sicca.

12. The composition for use according to claim 10 or 11, wherein the graft-versus-host disease is an acute graft-versus-host disease,
wherein
- the subject may have an acute graft-versus-host disease clinical grading of II, III or IV,
or wherein the graft-versus-host disease is a chronic graft-versus-host disease, wherein a global severity level of the chronic graft-versus-host disease may be
- moderate or severe.

13. The composition for use according to any one of the preceding claims, e.g., according to any one of claims 10 to 12, wherein the immune disorder and/or inflammatory disorder, e.g., the graft-versus-host disease, affects one or more of the following organs or tissues: skin, gut, intestine, liver, mucosa, mouth, eyes, lung, heart, and/or gastrointestinal system, e.g., skin, mucosa, mouth, eyes and/or lung.

14. The composition for use according to any one of the preceding claims, wherein the use in the prevention and/or treatment of the immune disorder and/or inflammatory disorder in the subject is a use in the prevention of a graft-versus-host disease in the subject.

15. A composition comprising
- platelet derived extracellular vesicles and at least one drug, wherein the at least one drug is selected from the group of immunosuppressive and/or anti-inflammatory drugs,
wherein the composition may preferably comprise
- platelet derived extracellular vesicles and at least two drugs, wherein at least one drug of the at least two drugs is selected from the group of immunosuppressive and/or anti-inflammatory drugs.
